(19) Europäisches Patentamt
European Patent Office
Office européen des brevets

(11) **EP 3 664 707 B1**

(12) **EUROPÄISCHE PATENTSCHRIFT**

(45) Veröffentlichungstag und Bekanntmachung des Hinweises auf die Patenterteilung:
**27.07.2022 Patentblatt 2022/30**

(21) Anmeldenummer: **18755739.2**

(22) Anmeldetag: **06.08.2018**

(51) Internationale Patentklassifikation (IPC):
***A61B 5/05*** *(2021.01)*   ***A61B 5/145*** *(2006.01)*
***A61B 5/00*** *(2006.01)*

(52) Gemeinsame Patentklassifikation (CPC):
**A61B 5/0507; A61B 5/145; A61B 5/14532;**
**A61B 5/7203;** A61B 5/681; A61B 2560/0223

(86) Internationale Anmeldenummer:
**PCT/EP2018/071280**

(87) Internationale Veröffentlichungsnummer:
**WO 2019/030178 (14.02.2019 Gazette 2019/07)**

(54) **ERFASSUNGSVORRICHTUNG UND -VERFAHREN UND COMPUTERPROGRAMM ZUM ERFASSEN EINER BLUTZUCKERKONZENTRATION**

DETECTION DEVICE AND METHOD, AND COMPUTER PROGRAM FOR DETECTING A BLOOD SUGAR CONCENTRATION

DISPOSITIF ET PROCÉDÉ DE DÉTECTION ET PROGRAMME INFORMATIQUE POUR DÉTECTER UNE CONCENTRATION DE GLUCOSE DANS LE SANG

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.08.2017 DE 102017118038**

(43) Veröffentlichungstag der Anmeldung:
**17.06.2020 Patentblatt 2020/25**

(73) Patentinhaber: **eesy-innovation GmbH**
**82008 Unterhaching (DE)**

(72) Erfinder: **FISCHER, Georg**
**90425 Nuernberg (DE)**

(74) Vertreter: **Bobbert & Partner**
**Patentanwälte PartmbB**
**Postfach 1252**
**85422 Erding (DE)**

(56) Entgegenhaltungen:
**EP-A1- 2 457 510      WO-A1-2012/069280**
**CN-A- 104 856 690      CN-A- 105 342 627**

## Beschreibung

TECHNISCHES GEBIET

Die vorliegende Erfindung betrifft das Gebiet der Erfassung einer Konzentration eines Blutbildparameters, beispielsweise einer Konzentration von Blutzucker. Insbesondere betrifft die vorliegende Erfindung eine Vorrichtung zur nicht-invasiven Erfassung des Blutbildparameters in einem Blutgefäß.

TECHNISCHER HINTERGRUND

Zur Bestimmung eines Blutbildparameters wie beispielsweise einer Konzentration eines Blutbestandteils kann invasiv Blut entnommen werden. Der Blutbildparameter kann dann unter Verwendung des entnommenen Bluts anhand standardisierter Teststreifen bestimmt werden, deren elektrische Widerstandswerte von der Konzentration des Blutbestandteiles, beispielsweise Blutzuckers, abhängen. Der jeweilige elektrische Widerstandswert kann beispielsweise unter Verwendung eines Blutzuckermessgerätes, das eine Gleichstrom-Widerstandsmessung zur Erfassung eines elektrischen Widerstandswertes eines Testreifens durchführt, erfasst werden. Der Widerstandswert kann anhand eines an sich bekannten Zusammenhanges zwischen einer Blutzuckerkonzentration und einem Widerstandswert in eine Blutzuckerkonzentration umgerechnet werden. Zur Erzielung einer hohen Erfassungsgenauigkeit ist jeder Teststreifen mit Kalibrierdaten, beispielsweise mit einem Referenzwiderstandswert oder mit einer entsprechenden Codierung, versehen, wodurch Variationen von Eigenschaften der Teststreifen ausgeglichen werden können. Nachteilig an invasiven Verfahren ist jedoch die Notwendigkeit der Blutentnahme und somit einer Verletzung eines Patienten. Außerdem ist eine kontinuierliche Erfassung einer Konzentration eines Blutbestandteils, um beispielsweise dessen Tagesgangkurve zu ermitteln, aufwändig. Darüber hinaus kann mittels des invasiven Verfahrens eine Zeitverzögerung zwischen einer Nahrungsaufnahme und beispielsweise einem Anstieg des Blutzuckers nicht genau erfasst werden. Ferner kann bei instantaner Kenntnis des Blutzuckerspiegels die Therapie durch Optimierung der Menge und des Zeitpunktes der Insulininjektion patientenindividuell und situationsbezogen angepasst werden.

Zur nichtinvasiven Bestimmung eines Blutbildparameters wie beispielsweise einer Stoffkonzentration oder einer Stoffzusammensetzung im Blut können mikrowellenspektroskopische Verfahren eingesetzt werden. Die Mikrowellenspektroskopie zur Erfassung von Blutbildparametern basiert auf einer Einkopplung eines Mikrowellensignals in Form einer elektromagnetischen Welle in ein blutdurchströmtes Gewebe oder ein blutdurchströmtes Gefäß und einer Erfassung einer frequenzabhängigen Absorption von der eingekoppelten Mikrowellenleistung.

In den Druckschriften von Buford Randal Jean et al., "A microwave frequency sensor for non-invasive blood-glucose measurement", SAS 2008 - IEEE Sensors Applications Symposium, Atlanta, GA, February 12 - 14, 2008, und von M. McClung, "Calibration methodology for a microwave non-invasive glucose sensor", Master's Thesis, Baylor University, Mai 2008, ist eine Elektrodenanordnung zur Bestimmung einer Blutzuckerkonzentration beschrieben. Dabei wird ausgenutzt, dass die dielektrischen Eigenschaften des Blutes vom Blutzuckergehalt abhängen. Durch Anpressen eines Daumens auf einen Mikrowellensensor wird eine Änderung der Dielektrizitätszahl des Daumens durch eine Verstimmung eines Resonators gemessen. Durch das Anpressen des Daumens wird jedoch Blut verdrängt, was zu einer Verfälschung der Messergebnisse führen kann. Des Weiteren können die Messungen nicht kontinuierlich durchgeführt werden. Die Auswertung der Messdaten zur Bestimmung des Blutzuckergehalts hängt zudem von dem jeweiligen Patienten ab, und ist daher bei anderen Patienten nicht reproduzierbar. Darüber hinaus ist mit diesem Verfahren die Eindringtiefe der Mikrowellenleistung nicht steuerbar, so dass eine Unterscheidung zwischen kapillarem und venösem Blut nicht möglich ist. Ferner wird die Änderung der Dielektrizitätszahl auf der Basis einer Eintormessung durchgeführt, welche anfällig bezüglich Fehlanpassungen ist.

Weiterhin ist die Signatur oder das Signal, welches benutzt wird, um beispielsweise die Blutzuckerkonzentration zu bestimmen, sehr schwach, und daher ist eine sorgfältige Kalibrierung des Mikrowellensensors oder einer Erfassungsvorrichtung zum Erfassen eines Blutbildparameters erforderlich. Bei verschiedenen Signalfrequenzen zeigen sich die sogenannten Relaxationsfrequenzen, welche für die im Blut vorhandenen Stoffe und ihre Konzentrationen typisch sind. Insbesondere zeigt sich der Glukosegehalt im Blut im Wesentlichen als Verschiebung der Relaxationsfrequenz von Wasser bei circa 20 GHz. Zudem wird die Frequenzverschiebung in der Signatur aber auch stark von der Temperatur beeinflusst, und die zu analysierenden Resonanzen zeigen sich als eine sehr kleine Änderung eines Transmissionsparameters (Streuparameter $S_{12}$) von nur 0,5 dB.

Um die Empfindlichkeit des Mikrowellensensors oder der Erfassungsvorrichtung zu erhöhen und Störungen zu eliminieren, können daher Differenzen ausgewertet werden, wobei zunächst eine Messung bei Glukosegehalt von 0 mg/dl und dann eine Messung bei dem zu bestimmenden Glukosegehalt von $x$ mg/dl durchgeführt wird. Von der bestimmten Transmissionskurve bei $x$ mg/dl wird die Transmissionskurve bei 0 mg/dl abgezogen. Die Verschiebung der Resonanz zeigt sich dann als typischer Doppelpuls, wobei dort, wo die Relaxationsfrequenz vorher war, ein negativer Puls ("Tal") entsteht, und dort, wo sie hingewandert ist, ein positiver Puls ("Berg").

Die Transmissionskurve bei 0 mg/dl dient als Referenzkurve, die immer wieder von der eigentlichen gemessenen Transmissionskurve abgezogen wird. Das konkrete Problem besteht nun darin, wie diese Referenzkurve praktisch in der Alltagsnutzung bestimmt werden kann. Ein Patient kann beispielsweise den Mikrowellensensor täglich einmal oder vor jeder Messung in eine Referenzflüssigkeit halten, um die Referenzkurve zu bestimmen. Über den Tag wird dann immer wieder eine Transmissionskurve gemessen, von der dann diese Referenzkurve abgezogen wird. Dieses Messverfahren hat jedoch mehrere Nachteile, da bei unsachgemäßer Kalibrierung die Messung falsch oder verfälscht sein könnte. Weiterhin könnte dies zu falschen Therapieentscheidungen führen, z. B. zu einer falschen Gabe der Menge an Insulin. Ferner ist eine Kalibrierung direkt vor jeder Messung nicht praktikabel, da für eine permanente Überwachung des Blutzuckergehalts eine Messung circa alle 15 Minuten (CGM, Continuous Glucose Measurement) stattfinden sollte.

Ferner können die Messungen durch eine Drift der Analogschaltungen verfälscht werden. Daher kann nicht davon ausgegangen werden, dass eine einmal durchgeführte Kalibrierung für 24 Stunden Bestand hat. Die Drift liegt eher im Minutenbereich und würde daher eine Kalibrierung vor jeder Messung erfordern. Es ist zudem den Patienten nicht zumutbar, Referenzflüssigkeiten für eine Kalibrierung permanent mit sich zu tragen.

Weiterhin besteht der große Wunsch bei CGM u.a. auch darin, ein Messsystem, welches eine einfache Bedienung ermöglicht, auch Kindern an die Hand zu geben.

Es besteht somit ein Bedarf, eine verbesserte Erfassungsvorrichtung zur nicht-invasiven Bestimmung eines Blutbildparameters, insbesondere einer Konzentration von Blutzucker, in einem durch ein Blutgefäß fließendes Blut zur Verfügung zu stellen, wobei keine Referenzflüssigkeitsmessungen benötigt sind, welche kurz vor der eigentlichen Glukosemessung für Kalibriermessungen herangezogen werden. Auch besteht ein Bedarf, eine verbesserte Erfassungsvorrichtung zur Verfügung zu stellen, welche keine aufwendige Interaktion durch den Patienten erfordert. Die Dokumente CN 104856690 A und CN105342627 A offenbaren als Stand der Technik Vorrichtungen und Methoden zur Blutzuckermessung mit Hilfe von Mikrowellen. Diese Dokumente offenbaren die Präambel der unabhängigen Ansprüche.

BESCHREIBUNG DER ERFINDUNG

Es ist daher die Aufgabe der vorliegenden Erfindung, eine verbesserte Erfassungsvorrichtung zur nicht-invasiven Bestimmung eines Blutbildparameters, insbesondere einer Konzentration von Blutzucker, in einem durch ein Blutgefäß fließenden Blut zur Verfügung zu stellen, wobei keine Referenzflüssigkeitsmessungen benötigt sind, welche kurz vor der eigentlichen Glukosemessung für Kalibriermessungen herangezogen werden. Auch besteht ein Bedarf, eine verbesserte Erfassungsvorrichtung zur Verfügung zu stellen, welche keine aufwendige Interaktion durch den Patienten erfordert.

Diese Aufgabe wird durch die Gegenstände der unabhängigen Ansprüche gelöst. Bevorzugte Ausführungsformen der Erfindung sind Gegenstand der abhängigen Ansprüche, der Beschreibung sowie der Figuren.

Die Erfindung basiert auf der Erkenntnis, dass ein Blutgefäß, wie beispielsweise eine Vene oder eine Arterie, zusammen mit das Blutgefäß umgebenden Fettgewebe sowie den darüber liegenden Hautschichten als ein dielektrischer Wellenleiter aufgefasst werden kann. Bei einer Anregung eines derartigen dielektrischen Wellenleitersystems können daher unterschiedliche Moden bzw. Wellentypen angeregt werden, beispielsweise eine transversalelektromagnetische (TEM) Welle oder transversal-elektrische (TE) Welle oder transversalmagnetische (TM) Welle oder ein hybrider Wellentyp wie eine HE-Welle, beispielsweise eine HE11- oder LP01-Welle. Bei einer TE-Welle existiert eine in Ausbreitungsrichtung weisende, von Null verschiedene Komponente des Magnetfeldes. Bei einer TM-Welle existiert hingegen eine in Modenausbreitungsrichtung weisende, von Null verschiedene Komponente eines elektrischen Feldes.

Gemäß einem ersten Aspekt betrifft die Erfindung eine Erfassungsvorrichtung nach Anspruch 1 zum Erfassen einer Blutzuckerkonzentration in einem Blutgefäß. Die Erfassungsvorrichtung umfasst einen Signalgenerator, welcher ausgebildet ist, ein Kalibrierungsmesssignal zu erzeugen, wobei das Kalibrierungsmesssignal eine Überlagerung aus einem ersten elektrischen Anregungssignal und einem zweiten Anregungssignal umfasst, wobei das erste elektrische Anregungssignal eine höhere Frequenz als das zweite Anregungssignal aufweist und/oder das zweite Anregungssignal ein Gleichsignal ist und/oder wobei das zweite Anregungssignal eine höhere Leistung als das erste Anregungssignal aufweist. Die Erfassungsvorrichtung umfasst gemäß einem ersten Aspekt zudem eine Sende-Empfangsanordnung, welche ausgebildet ist, das Kalibrierungsmesssignal in Richtung des Blutgefäßes auszusenden und ansprechend auf das Aussenden des Kalibrierungssignals ein erstes Systemantwortsignal zu empfangen, wobei die Sende-Empfangsanordnung ausgebildet ist, ein drittes Anregungssignal in Richtung des Blutgefäßes auszusenden, und ansprechend auf das Aussenden des dritten Anregungssignals ein zweites Systemantwortsignal zu empfangen, wobei die Erfassungseinrichtung zudem einen Prozessor umfasst, welcher ausgebildet ist, das erste Systemantwortsignal und das zweite Systemantwortsignal zu verknüpfen, um ein Messsignal zur Bestimmung des Blutbildparameters zu erhalten. Der Blutbildparameter umfasst eine Zuckerkonzentration bzw. Glukosekonzentration des Blutes in dem Blutgefäß. Das erste elektrische Anregungssignal und das dritte Anregungssignal können Mikrowellensignale sein. Das zweite Anregungssignal kann ein Gleichsignal (DC) oder ein niederfrequentes Wechselsignal (AC) sein.

Die vorliegend beschriebenen Anregungssignale sind elektrische Signale. Die Leistung des jeweiligen Signals kann eine elektrische Leistung oder eine elektromagnetische Leistung sein.

Die vorstehend beschriebenen Signale, insbesondere die Anregungssignale, Gleichsignale und Wechselsignale, können beispielsweise elektrische Ströme, elektrische Spannungen, magnetische und/oder elektrische Felder sein. Das Gleichsignal weist dabei vorzugsweise über die Zeit keine Polaritätswechsel auf, besonders vorzugsweise beschreibt das Gleichsignal ein Signal konstanter Größe, beispielsweise eine Gleichspannung, einen Gleichstrom, ein konstantes elektrisches Feld und/oder ein konstantes magnetisches Feld. Das Wechselsignal kann beispielsweise eine Wechselspannung, ein Wechselstrom und/oder ein elektromagnetisches Wechselfeld sein.

Durch die Erfassungsvorrichtung gemäß dem ersten Aspekt wird beispielsweise der technische Vorteil erreicht, dass ein Kalibrierungsmesssignal erzeugt wird, welches keine Referenzflüssigkeiten benötigt, welches kurz vor der eigentlichen Glukosemessung in dem Blut erzeugt werden kann, und welches keine aufwendige Interaktion durch den Patienten erfordert. Weiterhin wird dadurch der Vorteil erreicht, dass das Messsignal zur Bestimmung des Blutbildparameters nicht-invasiv erhalten werden kann.

Ein Blutbildparameter im Sinne der vorliegenden Erfindung ist beispielsweise eine Kennzahl, ein Parameterwert und/oder die Ausprägung eines Parameters im Blut eines Patienten. Ein Patient kann dabei ein Mensch sein oder ein Tier. Ein Patient kann gesund oder krank sein. Basierend auf dem gemessenen Blutbildparameter einerseits und den gesamten übrigen Umständen andererseits kann ein Arzt eine vorliegende Funktionsstörung und/oder Krankheit diagnostizieren.

Aus der Praxis sind Verfahren zum Bestimmen der Glukosetoleranz und/oder der Insulinresistenz einer Person bekannt. Diese Verfahren dienen der Erkennung und Differenzierung einer gestörten Glukosehomöostase ("impaired fastening glucose"), bzw. eines Diabetes mellitus. Hierzu werden zunächst Blutparameter des Betroffenen bestimmt. Basierend auf den gemessenen Blutparameterwerten einerseits, und den gesamten Umständen, welche die gemessenen Werte mit beeinflusst haben können andererseits, kann der Arzt eine ggf. vorliegende gestörte Glukosehomöostase diagnostizieren.

Zu den Umständen, welche zum Stellen einer solchen Diagnose zwingend zu beachten sind, zählt u. a., ob der Patient bei Abnahme der Blutzuckerwerte nüchtern oder post-prandial gewesen ist, wieviel Zeit seit einer Test-Glukosegabe vergangen ist, ob der Patient Fieber hat, ob er in den vergangenen Tagen eine Mindestmenge an Kohlenhydraten zu sich genommen hat und ob der Patient unter Medikation steht.

Im Falle einer Medikamenteneinnahme sind insbesondere solche Medikamente relevant, welche den Blutzuckerspiegel beeinflussen können. Zu diesen Medikamenten zählen bspw. Kortikoide, Kontrazeptiva oder orale Antidiabetika.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wenn hierin von "ausgebildet", "programmiert" oder "konfiguriert" die Rede ist, so ist auch offenbart, diese Begriffe gegeneinander auszutauschen.

Wann immer hierin eine Eignung oder ein Verfahrensschritt genannt ist, umfasst die vorliegende Erfindung auch eine entsprechende Programmierung oder Konfigurierung einer geeigneten Vorrichtung oder eines Abschnitts hiervon sowie derart programmierte Vorrichtungen.

Vorteilhafte Weiterentwicklungen der vorliegenden Erfindung sind jeweils Gegenstand von Unteransprüchen und Ausführungsformen.

Wenn hierin von einer Ausführungsform die Rede ist, so stellt dies eine erfindungsgemäße, beispielhafte Ausführungsform dar.

Wann immer Verfahren hierin offenbart sind, so umfasst die Offenbarung zugleich Vorrichtungen, insbesondere mit einer dem Verfahren entsprechenden Bezeichnung, die zur Ausführung des Verfahrens geeignet, insbesondere konfiguriert, sind.

Erfindungsgemäße Ausführungsformen können eines oder mehrere der oben und/oder im Folgenden genannten Merkmale in jeder technisch möglichen Kombination aufweisen.

Hier werden in einigen Fällen die Begriffe Antenne, Antennenstruktur, Koppelvorrichtung, Koppelstruktur und Elektrode synonym verwendet.

In einer Ausführungsform der Erfassungsvorrichtung gemäß dem ersten Aspekt umfasst die Sende-Empfangsanordnung eine Antennenanordnung, welche ausgebildet ist, das elektromagnetische Feld des Kalibrierungsmesssignals oder des dritten Anregungssignals auszusenden. Die Antennenanordnung kann auch elektromagnetische Ankopplung genannt werden.

In einer Ausführungsform der Erfassungsvorrichtung gemäß dem ersten Aspekt umfasst die Antennenanordnung eine elektrische Leitungsanordnung, einen halboffenen Wellenleiter, insbesondere einen geschlitzten Hohlleiter, oder eine Mikrostreifenleitung.

Eine dielektrische Belastung einer angeregten elektrischen Leitungsanordnung in der unmittelbaren Nähe zu einem Blutgefäß kann zu einer Änderung des elektromagnetischen Feldes der elektrischen Leitungsanordnung führen. Eine Erfassung dieser Änderung kann für die Bestimmung eines Blutbildparameters ausgenutzt werden, da sich Veränderungen des elektromagnetischen Feldes oberhalb der Leitung auf Veränderungen der elektrischen Leitungseigenschaften abbilden. Der zu bestimmende Blutbildparameter ist eine Konzentration von Blutzucker im Blut, so ist eine Änderung des elektromagnetischen Feldes der elektrischen Leitungsanordnung ein Maß für die Konzentration des Blutzuckers, d. h. für den Blutzuckerspiegel. Die Erfassung des Blutbildparameters auf der Basis der Änderung des elektromagnetischen Feldes basiert auf der weiteren Erkenntnis, dass sich die Viskosität einer Wasserlösung mit zunehmender Zuckerkonzentration ändern kann und sich damit auf das frequenzabhängige Transmissionsverhalten der elektrischen Leitungsanordnung auswirkt. Auf diese Weise kann durch eine Erfassung einer Änderung des elektromagnetischen Feldes und damit der elektrischen Leitungseigenschaften, beispielsweise durch eine Streuparametermessung (S-Parameter) in einem Frequenzbereich von beispielsweise bis zu 100 GHz, insbesondere 1 MHz bis 100 GHz, die Konzentration des Blutzuckers erfasst werden.

In Abhängigkeit von einer Mikrowellenanregung durch die elektrische Leitungsanordnung können in einem das Blutgefäß und die Hautschichten umfassenden dielektrischen Wellenleitersystem unterschiedliche, auch in Blutflussrichtung ausbreitungsfähige Moden angeregt werden, welche zu einer Belastung der elektrischen Leitungsanordnung führen, wodurch eine genaue Erfassung eines Blutbildparameters mittels einer Erfassungsvorrichtung möglich ist.

In einer Ausführungsform der Erfassungsvorrichtung gemäß dem ersten Aspekt mit einem Armband, ist zumindest die Sende-Empfangsanordnung oder die Erfassungsvorrichtung in dem Armband integriert. In weiteren Ausführungsformen ist die Erfassungsvorrichtung ausgestaltet als eine Vorrichtung zur Ankopplung an einen Finger, an ein Ohrläppchen und/oder an eine Zunge (beispielsweise in Anlehnung an einen Lutscher) oder enthält eine solche Vorrichtung.

In einer Ausführungsform der Erfassungsvorrichtung gemäß dem ersten Aspekt ist der Prozessor ausgebildet, eine Differenz aus dem ersten Systemantwortsignal und dem zweiten Systemantwortsignal zu bilden, um das Messsignal zu erhalten.

Durch die Auswertung von Differenzen wird der Vorteil erreicht, dass die Empfindlichkeit der Erfassungsvorrichtung erhöht wird, und Störungen im Wesentlichen eliminiert werden können. Weiterhin wird dadurch der Vorteil erreicht, dass das Messsignal sehr genau bestimmt werden kann.

In einer Ausführungsform der Erfassungsvorrichtung gemäß dem ersten Aspekt ist der Signalgenerator ausgebildet, das erste elektrische Anregungssignal als ein Kleinsignal und das zweite Anregungssignal als ein Großsignal zu erzeugen.

Im Sinne der Erfindung handelt es sich sowohl bei dem Kleinsignal als auch bei dem Großsignal um Signale, wobei mit diesen Signalen nicht notwendigerweise absolute oder relative Leistungswerte verbunden sind. Vorzugsweise weist das Großsignal eine größere Amplitude als das Kleinsignal auf.

Dadurch wird beispielsweise der Vorteil erreicht, dass durch das Großsignal die Dipole in dem Blut, welche durch das Kleinsignal hin- und herrotiert werden, daran gehindert werden, mit zu rotieren (zu relaxieren). Damit wird eine Relaxation von den im Blut vorhandenen Stoffen und ihren Konzentrationen im Wesentlichen ausgeschaltet.

In einer Ausführungsform der Erfassungsvorrichtung gemäß dem ersten Aspekt ist der Signalgenerator ausgebildet, das erste elektrische Anregungssignal als ein schwaches hochfrequentes Signal und das zweite Anregungssignal als ein niederfrequentes starkes Signal, insbesondere als ein Gleichsignal, zu erzeugen.

Der Signalgenerator ist ausgebildet das erste elektrische Anregungssignal mit einer Frequenz zwischen 1 MHz bis 100 GHz, zu erzeugen.

Simulationen haben gezeigt, dass eine Blutader als quasi dielektrischer Wellenleiter sehr hohe Verluste aufweist, die Wellenführung an sich aber funktioniert. Der Hintergrund ist die Leitfähigkeit des Blutes. Wegen dieses Effektes steigen auch die Verluste zu hohen Frequenzen stark an. Dies bedeutet aber andererseits, dass eine Messung im hohen Frequenzbereich sehr genaue Aufschlüsse über die Beschaffenheit des Bluts liefert. So können bei Frequenzen im Gigahertzbereich die Streuparameter besonders genau bestimmt werden. Insbesondere haben Simulationen gezeigt, dass Änderungen der Permittivität und der magnetischen Permeabilität in einem höheren Gigahertzbereich von etwa 20 bis etwa 60 GHz deutlich ausgeprägter sind als in einem unteren Gigaherzbereich von 1 bis 15 GHz. Mit steigender Frequenz reagiert zudem die Phase der Streuparameter immer empfindlicher. Daher eignet sich insbesondere eine Messung im höheren Gigahertzbereich, um den gewünschten Blutbildparameter sehr präzise zu erfassen.

In einer Ausführungsform der Erfassungsvorrichtung gemäß dem ersten Aspekt ist der Signalgenerator ausgebildet, das erste elektrische Anregungssignal dem zweiten Anregungssignal zu überlagern, um das Kalibrierungsmesssignal zu erzeugen.

In einer Ausführungsform der Erfassungsvorrichtung gemäß dem ersten Aspekt ist die Sende-Empfangsanordnung ausgebildet, das erste Systemantwortsignal und das zweite Systemantwortsignal durch eine S-Parametermessung, insbesondere einer Transmissionsmessung und/oder einer Reflexionsmessung, zu erfassen.

Das liegende Blutgefäß kann als ein dielektrischer Wellenleiter interpretiert werden, in dem je nach Frequenz verschiedene Moden bzw. Wellentypen, beispielsweise eine TE-Welle, eine TM-Welle, eine TEM-Welle oder eine HE-Welle, ausbreitungsfähig sein können. Wegen der hohen Verluste können die Moden degradiert sein. Die Sende-Empfangsanordnung kann derart ausgestaltet sein, dass die elektrische Leitungsanordnung anregt wird, um dezidiert eine Mikrowellenleistung in das Blutgefäß einzukoppeln, und diese beispielsweise nach einigen Millimetern bis einigen Zentimetern wieder auszukoppeln. Die elektrische Leitungsanordnung kann zu diesem Zweck als Mikrostreifenleitung ausgestaltet sein, so dass ein Leitungsstreifen als Erreger zur Einkopplung von Mikrowellenenergie und der andere Leitungsstreifen als Empfänger zum Auskoppeln der Mikrowellenenergie dient. Das Blutgefäß dient dabei als eine Messstrecke, welche gleichzeitig jedoch eine Last für die Mikrowellenenergie darstellt und die Ausprägung der elektromagnetischen Feldlinien und/oder die Ausbreitung der elektromagnetischen Wellen beeinflusst, insbesondere beeinträchtigt. Das Blutgefäß ist als ein verteiltes Element und nicht mehr als ein konzentriertes Element anzusehen. Alternativ kann auch eine Leitung mit zwei Toren benutzt werden, wobei die elektromagnetische Feldenergie zum Teil innerhalb des Mikrostreifenleiters und zum anderen in dem durch das Blutgefäß und seine Umgebung gebildeten dielektrischen Wellenleiter angesiedelt ist. Das nicht innerhalb der Leitung geführte elektromagnetische Feld interagiert mit dem Blut. Dadurch werden die Streuparameter der Leitung abhängig von den Bluteigenschaften.

Bevorzugt wird die Messung der Feldänderung auf der Basis einer Zweitormessung durchgeführt. Dabei können Primärmoden in dem dielektrischen Wellenleiter, d. h. dem Blutgefäß, angeregt werden, so dass mit einer derartigen Messmethode die Erfassung des Blutbildparameters sehr präzise vorgenommen werden kann. Um Primärmoden im dielektrischen Wellenleitersystem anzuregen, kann berücksichtigt werden, dass je nach gewählter Frequenz eines Anregungssignals unterschiedliche Moden dominant sein können. Bevorzugt sind Modentypen, die eine Konzentration der Felder in dem Blutgefäß aufweisen, solchen Moden vorzuziehen, bei denen die Felder in einer Hautschicht konzentriert sind. Aufgrund der dielektrischen Eigenschaften des Blutgefäßes zeigt sich, dass für bestimmte Modentypen longitudinale Komponenten $E_{longitudinal}$, $H_{longitudinal}$ in Ausbreitungsrichtung, d. h. in Richtung eines Blutgefäßverlaufs, stärker als die transversalen Komponenten $E_{transversal}$, $H_{transversal}$, d. h. quer zum Blutgefäßverlauf, sind. Bevorzugt werden daher in dem zu erfassenden Frequenzbereich diejenigen Moden angeregt, welche eine maximale Einkopplung der Mikrowellenleistung in das Blutgefäß ermöglichen. Eine S-Parametermessung eignet sich besonders gut, die Änderungen des elektromagnetischen Feldes aufgrund der Belastung mit dem Blutgefäß zu messen.

In einer Ausführungsform der Erfassungsvorrichtung gemäß dem ersten Aspekt ist der Signalgenerator ausgebildet, das erste Systemantwortsignal und das zweite Systemantwortsignal basierend auf einer Messung eines Vorwärts-Transmissionsfaktors ($S_{21}$) und/oder eines Eingangs-Reflexionsfaktors ($S_{11}$) zu bestimmen.

Vorfeldmessungen haben gezeigt, dass eine Änderung der Zuckerkonzentration signifikante Einflüsse sowohl auf den Vorwärts-Transmissionsfaktor $S_{21}$ als auch auf den Eingangs-Reflexionsfaktor $S_{11}$ hat. Zur Bestimmung der beiden Streuparameter $S_{21}$ und $S_{11}$ kann die Sende-Empfangsanordnung einen Netzwerkanalysator, beispielsweise einen vektoriellen oder einen skalaren Netzwerkanalysator, umfassen.

In einer Ausführungsform der Erfassungsvorrichtung gemäß dem ersten Aspekt ist der Prozessor ausgebildet, den Blutbildparameter auf der Basis des Messsignals zu bestimmen.

In einer Ausführungsform der Erfassungsvorrichtung gemäß dem ersten Aspekt ist der Prozessor oder die Sende-Empfangsanordnung ausgebildet, zur Bestimmung des Blutbildparameters eine komplexe Dielektrizitätszahl $\varepsilon$ zu bestimmen, wobei der Realteil $\varepsilon'$ der komplexen Dielektrizitätszahl $\varepsilon$ im Wesentlichen die Polarisierbarkeit eines Stoffes im Blut abbildet, und der Imaginärteil $\varepsilon''$ dessen Verluste.

Der Realteil $\varepsilon'$ der komplexen Dielektrizitätszahl $\varepsilon$ beschreibt die dielektrische Leitfähigkeit, auch Permittivität oder $\varepsilon_r$ genannt, eines Materiales, da die Dielektrizitätszahl als das dimensionslose Verhältnis der Permittivität des Materiales zur Permittivität des Vakuums definiert ist.

Insbesondere ist die Permittivität ein charakteristischer Parameter für die Durchlässigkeit des Materials für elektrische Felder und kann daher verwendet werden, um den Blutbildparameter zu charakterisieren. Beispielsweise ändert sich die Permittivität von Blut abhängig von der Zuckerkonzentration, so dass von der Permittivität auf die Zuckerkonzentration geschlossen werden kann.

In einer Ausführungsform der Erfassungsvorrichtung gemäß dem ersten Aspekt ist der Prozessor oder die Sende-Empfangsanordnung ausgebildet, zur Bestimmung des Blutbildparameters eine Relaxationszeitkonstante ($\tau$) auf der Basis der Formel:

$$\tau = \frac{1}{2\pi f_A},$$

zu berechnen, wobei $f_A$ eine Relaxationsfrequenz bezeichnet, bei welcher der Imaginärteil $\varepsilon''$ der komplexen Dielektrizitätszahl maximal ist, und wobei der Prozessor oder die Sende-Empfangsanordnung ausgebildet ist, den Blutbildparameter, beispielsweise die Glukosekonzentration im Blut, in Abhängigkeit von der bestimmten Relaxationszeitkonstanten $\tau$ zu ermitteln.

Der Signalgenerator ist ausgebildet ein Löschsignal, insbesondere ein stationäres oder abklingendes Wechselsignal, zum Löschen einer durch das Kalibrierungssignal verursachten Polarisation von Dipolen in dem Blutgefäß zu erzeugen, wobei die Sende-Empfangsanordnung ausgebildet ist, das Löschsignal in Richtung des Blutgefäßes auszusenden.

In einer Ausführungsform der Erfassungsvorrichtung gemäß dem ersten Aspekt ist die Sende-Empfangsanordnung ausgebildet, das Löschsignal vor oder nach dem Aussenden des Kalibrierungsmesssignals oder nach dem Aussenden des dritten Anregungssignals auszusenden. Dadurch wird die identische Ausrichtung der dielektrischen Dipole im Blut, welche durch einen Gleichsignalanteil in dem Messsignal entstehen kann, wieder rückgängig gemacht. Die Ausrichtung der Dipole wird in den normalen ungeordneten Zustand zurückgeführt.

In einer Ausführungsform wird nach dem Kalibriermesssignal gelöscht, weil dann alle Dipole ausgerichtet sind. Durch die Löschung wird wieder eine normale Unordnung der Dipole bewirkt.

Man kann die Reihenfolge auch umdrehen und zunächst normal messen, beispielsweise ohne Großsignal bzw. DC, und dann die Kalibriermessung (beispielsweise inkl. Großsignal bzw. DC) durchführen. Die Reihenfolge dieser Schritte kann daher beliebig sein.

Gemäß einem zweiten Aspekt betrifft die Erfindung ein Verfahren nach Anspruch 16 zum Erfassen einer Blutzuckerkonzentration in einem Blutgefäß. Das Verfahren umfasst die folgenden Schritte: Erzeugen eines Kalibrierungsmesssignals, wobei das Kalibrierungsmesssignal eine Überlagerung aus einem ersten elektrischen Anregungssignal und einem zweiten Anregungssignal umfasst, wobei das erste Anregungssignal eine höhere Frequenz als das zweite Anregungssignal aufweist und/oder das zweite Anregungssignal ein Gleichsignal ist und/oder wobei das zweite Anregungssignal eine höhere elektrische Leistung als das erste elektrische Anregungssignal aufweist, Aussenden des Kalibrierungsmesssignals in Richtung des Blutgefäßes, Empfangen eines ersten Systemantwortsignals ansprechend auf das Aussenden des Kalibrierungssignals, Aussenden eines Löschsignals, Aussenden eines dritten Anregungssignals in Richtung des Blutgefäßes, Empfangen eines zweiten Systemantwortsignals ansprechend auf das Aussenden des dritten Anregungssignals, Verknüpfen des ersten Systemantwortsignals und des zweiten Systemantwortsignals, und Erhalten eines Messsignals zur Bestimmung des Blutbildparameters. Das Messsignal kann beispielsweise durch Differenzbildung, insbesondere der Systemantwortsignale, erzeugt werden.

Weiters umfasst das Verfahren den folgenden Schritt: Aussenden eines Löschsignals vor dem Aussenden des Kalibrierungsmesssignals oder nach dem Aussenden des dritten Anregungssignals.

Die Reihenfolge der Verfahrensschritte kann in einer Ausführungsform beliebig gewählt werden.

Das Verfahren gemäß dem zweiten Aspekt kann in einer Ausführungsform durch die Erfassungsvorrichtung gemäß dem ersten Aspekt ausgeführt werden.

Gemäß einem dritten Aspekt betrifft die Erfindung ein Computerprogramm mit einem Programmcode zum Ausführen des Verfahrens nach dem zweiten Aspekt, wenn der Programmcode auf einer hierzu geeigneten Erfassungsvorrichtung ausgeführt wird. In einer Ausführungsform umfasst die Erfindung ein digitales, insbesondere nicht-flüchtiges, Speichermedium (hier auch als Träger bezeichnet), insbesondere in Form von Diskette, RAM, ROM, CD, Festplatte, DVD, USB-Stick, Flashcard, SD-Karte oder EPROM, insbesondere mit elektronisch oder optisch auslesbaren Steuersignalen, das derart konfiguriert sein kann, um das Verfahren nach Anspruch 16 auf einer Erfassungsvorrichtung auszuführen.

Dabei können alle, einige oder manche der maschinell durchführbaren Schritte dieses Verfahrens veranlasst werden.

Ein erfindungsgemäßes Computerprogramm-Produkt weist einen volatilen, flüchtigen oder auf einem maschinenlesbaren Träger gespeicherten Programmcode auf, durch den eine hierzu geeignete Erfassungsvorrichtung das Verfahren nach Anspruch 16 ausführen kann.

Dabei können alle, einige oder manche der maschinell durchführbaren Schritte dieses Verfahrens veranlasst werden.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte, ein EPROM und dergleichen sein.

Ein erfindungsgemäßes Computerprogramm weist einen Programmcode auf, durch den eine hierzu geeignete Erfassungsvorrichtung das Verfahren nach Anspruch 16 ausführen kann.

Dabei können alle, einige oder manche der maschinell durchführbaren Schritte dieses Verfahrens veranlasst werden.

Unter einem Computerprogramm-Produkt kann erfindungsgemäß beispielsweise ein auf einem Träger gespeichertes Computerprogramm, ein Embedded System als umfassendes System mit einem Computerprogramm (z. B. ein elektronisches Gerät mit einem Computerprogramm), ein Netzwerk von computerimplementierten Computerprogrammen (z. B. Client/Serversystem, Cloud Computing System, etc.), oder ein Computer, auf dem ein Computerprogramm geladen ist, abläuft, gespeichert ist, ausgeführt oder entwickelt wird, verstanden werden.

Unter einem Computerprogramm kann erfindungsgemäß beispielsweise ein physikalisches, vertriebsfähiges Software-Produkt verstanden werden, welches ein Programm aufweist. Ferner offenbart wird eine Erfassungsvorrichtung zum Erfassen eines Blutbildparameters in einem Blutgefäß, mit einem Signalgenerator, welcher ausgebildet ist, ein Anregungssignal zu erzeugen, einer Sende-Empfangsanordnung, welche ausgebildet ist, das Anregungssignal in Richtung des Blutgefäßes auszusenden, und ansprechend auf das Aussenden des Anregungssignal ein Systemantwortsignal zu empfangen, und einem Prozessor, welcher ausgebildet ist, den Blutbildparameter auf der Basis des Systemantwortsignals zu bestimmen, wobei der Signalgenerator ausgebildet ist, ein Löschsignal, insbesondere ein stationäres oder abklingendes Wechselsignal, zum Löschen einer durch das Anregungssignal verursachten Polarisation von Dipolen in dem Blutgefäß zu erzeugen, wobei die Sende-Empfangsanordnung ausgebildet ist, das Löschsignal in Richtung des Blutgefäßes auszusenden.

Das Anregungssignal kann einer Überlagerung des ersten elektrischen Anregungssignals mit dem zweiten Anregungssignal gemäß dem ersten Aspekt oder dem dritten Anregungssignal gemäß dem ersten Aspekt entsprechen.

Dadurch kann die dielektrische Polarisation von Dipolen im Blut, welche durch einen möglichen Gleichsignalanteil in dem Anregungssignal entstehen kann, wieder rückgängig gemacht werden.

Die Bestimmung des Blutbildparameters kann wie im Zusammenhang mit einem der vorgenannten Aspekte beschrieben erfolgen.

Dabei kann die Sende-Empfangsanordnung ausgebildet sein, das Löschsignal vor dem Aussenden des Anregungssignals oder nach dem Aussenden des Anregungssignals auszusenden.

Dabei kann die Sende-Empfangsanordnung eine elektromagnetische Ankopplungsstruktur umfassen, welche ausgebildet ist, das elektromagnetische Feld des dritten Anregungssignals auszusenden.

Dabei kann die Antennenanordnung eine elektrische Leitungsanordnung oder einen halboffenen Wellenleiter, insbesondere einen geschlitzten Hohlleiter, oder eine Mikrostreifenleitung umfassen.

Dabei kann die Erfassungsvorrichtung ein Armband umfassen, wobei zumindest die Sende-Empfangsanordnung oder die Erfassungsvorrichtung in dem Armband integriert ist.

Dabei kann der Signalgenerator ausgebildet sein, das Anregungssignal aus einer Überlagerung eines elektrischen Wechselsignals und eines elektrischen Gleichsignals zu erzeugen, wobei das elektrische Gleichsignal eine höhere elektrische Leistung als das elektrische Wechselsignal aufweist.

Das Anregungssignal kann einer Überlagerung des ersten elektrischen Anregungssignals mit dem zweiten Anregungssignal gemäß dem ersten Aspekt oder dem dritten Anregungssignal gemäß dem ersten Aspekt entsprechen.

Dabei kann der Signalgenerator ausgebildet sein, das Anregungssignal mit einer Frequenz bis 100 GHz, insbesondere zwischen 1 MHz und 100 GHz, zu erzeugen.

Dabei kann die Sende-Empfangsanordnung ausgebildet sein, das Systemantwortsignal durch eine S-Parametermessung, insbesondere eine Transmissionsmessung und/oder eine Reflexionsmessung, zu erfassen.

Dabei kann der Signalgenerator ausgebildet sein, das Systemantwortsignal basierend auf einer Messung eines Vorwärts-Transmissionsfaktors $(S_{21})$ und/oder eines Eingangs-Reflexionsfaktors $(S_{11})$ zu bestimmen.

Dabei kann der Prozessor ausgebildet sein, den Blutbildparameter auf der Basis des Systemantwortsignals zu bestimmen.

Dabei kann der Prozessor oder die Sende-Empfangsanordnung ausgebildet sein, zur Bestimmung des Blutbildparameters eine komplexen Dielektrizitätszahl $\varepsilon$ zu bestimmen, wobei der Realteil $\varepsilon'$ der komplexen Dielektrizitätszahl $\varepsilon$ im Wesentlichen die Polarisierbarkeit eines Stoffes im Blut abbildet und der Imaginärteil $\varepsilon''$ dessen Verluste.

Weiter wird offenbart ein Verfahren zum Erfassen eines Blutbildparameters in einem Blutgefäß, mit: Erzeugen eines Anregungssignals (insbesondere mit einem Gleichanteil), insbesondere mittels eines Signalgenerators, Aussenden des Anregungssignals in Richtung des Blutgefäßes und ansprechend auf das Aussenden des Messsignals, Empfangen eines Systemantwortsignals, insbesondere mittels einer Sende-Empfangsanordnung, Bestimmen des Blutbildparameters auf der Basis des Systemantwortsignals, Erzeugen eines Löschsignals, insbesondere eines stationären oder abklingenden Wechselsignals, zum Löschen einer durch das Anregungssignal verursachten Polarisation von Dipolen in dem Blutgefäß, insbesondere mittels des Signalgenerators, und Aussenden des Löschsignals in Richtung des Blutgefäßes, insbesondere mittels der Sende-Empfangsanordnung.

Dadurch kann die identische Ausrichtung der elektrischen Dipole im Blut, welche durch einen möglichen Gleichsignalanteil in dem Anregungssignal entstehen kann, wieder rückgängig gemacht werden.

Die Bestimmung des Blutbildparameters kann wie im Zusammenhang mit einem der vorgenannten Aspekte beschrieben erfolgen.

Das Anregungssignal kann einer Überlagerung des ersten elektrischen Anregungssignals mit dem zweiten Anregungssignal gemäß dem ersten Aspekt oder dem dritten Anregungssignal gemäß dem ersten Aspekt entsprechen.

Dabei kann das Verfahren in einer Ausführungsform durch die Erfassungsvorrichtung gemäß dem vierten Aspekt ausgeführt werden.

Weitere Merkmale entsprechen den Merkmalen der Erfindung gemäß den vorgenannten Aspekten und werden im Zusammenhang mit diesen beschrieben.

In einigen Ausführungsformen der Erfindung ist das erste Anregungssignal und/oder das dritte Anregungssignal in einem Bereich von 18 bis 25 GHz, vorzugsweise in einem Bereich von 18 bis 20 GHz, besonders vorzugsweise bei etwa 19, etwa 20 und/oder etwa 24 GHz.

In einigen Ausführungsformen der Erfindung ist das erste Anregungssignal und/oder das dritte Anregungssignal in einem Bereich von 50 bis 60 GHz, vorzugsweise von 53 bis 55 GHz, besonders vorzugsweise bei etwa 54 GHz.

In einigen Ausführungsformen der Erfindung hat das erste Anregungssignal und/oder das dritte Anregungssignal eine Frequenz von weniger als 93 GHz, vorzugsweise weniger als 60 GHz, vorzugsweise weniger als 52 GHz, vorzugsweise weniger als 50 GHz, vorzugsweise weniger als 40 GHz, besonders vorzugsweise weniger als 30 GHz. In einigen Ausführungsformen ist das erste und/oder dritte Anregungssignal nicht in einem Bereich von 50 bis 75 GHz, vorzugsweise nicht in einem Bereich von 57 bis 64 GHz, besonders vorzugsweise nicht in einem Bereich von 18 bis 25 GHz.

In einigen Ausführungsformen hat das niederfrequente Wechselsignal eine Frequenz von weniger als 30 kHz, vorzugsweise weniger als 3 kHz, besonders vorzugsweise weniger als 300 Hz und ganz besonders vorzugsweise weniger als 30 Hz.

In einigen Ausführungsformen erfordert das Verfahren ein Aussenden des ersten, zweiten und/oder dritten Anregungssignals in unmittelbarer Nähe der Haut, insbesondere eines Blutgefäßes, vorzugsweise mit einem Teil der Sendeeinrichtung in unmittelbarem Kontakt mit der Haut.

In einigen Ausführungsformen verwenden Sende- und/oder Empfangsstrukturen der Erfassungsvorrichtung nicht die Modulationstechnik frequency modulated continuous wave (FMCW) und/oder verwenden einige oder alle der in dem erfindungsgemäßen Verfahren ausgesendeten Signale nicht diese Modulationstechnik.

Beispielsweise können als Teil des erfindungsgemäßen Verfahrens z. B. breitbandige Code-Sequenzen ausgesendet werden.

In einigen Ausführungsformen erfordert das Verfahren keine unmittelbare Nähe der Sendeeinrichtung, insbesondere einer Koppelstruktur der Sendeeinrichtung, zu Haut und/oder Blutgefäß. In einigen Ausführungsformen kann die Erfassungseinrichtung zur Messung mehr als 0,5 mm, vorzugsweise mehr als 2 mm, besonders vorzugsweise mehr als 5 mm und ganz besonders vorzugsweise mehr als 10 mm von Haut und/oder Blutgefäß entfernt sein und dennoch den Blutbildparameter, insbesondere Glukose, messen.

In einigen Ausführungsformen verwendet das erfindungsgemäße Verfahren Radar, um Blutbildparameter insbesondere aus der Entfernung (mehr als 1 mm, vorzugsweise mehr als 3 mm, besonders vorzugsweise mehr als 5 mm) zu bestimmen. In anderen Ausführungsformen wird Radar nicht verwendet.

In einigen Ausführungsformen ist die Leistung des zweiten Anregungssignals mindestens 5-mal, vorzugsweise mindestens 10-mal, besonders vorzugsweise mindestens 100-mal und ganz besonders vorzugsweise mindestens 1000-mal so groß wie die Leistung des ersten Anregungssignals.

In einigen Ausführungsformen ist die Leistung des zweiten Anregungssignals höchstens 10-mal, vorzugsweise höchstens 100-mal, besonders vorzugsweise höchstens 1000-mal und ganz besonders vorzugsweise höchstens 10000-mal so groß wie die Leistung des ersten Anregungssignals.

In einigen Ausführungsformen umfasst das Kalibrierungsmesssignal ausschließlich das erste und das zweite Anregungssignal, in weiteren Ausführungsformen umfasst das Kalibrierungsmesssignal zusätzlich ein oder mehrere weitere Anregungssignale.

In einigen Ausführungsformen umfasst der Verfahrensschritt des Aussendens des dritten Anregungssignals auch das Aussenden eines oder mehrerer zusätzlicher Signale, in anderen Ausführungsformen ist dies nicht der Fall.

In einigen Ausführungsformen stellt das erste Anregungssignal ein Kleinsignal und das zweite Anregungssignal ein Großsignal dar. Das zweite Anregungssignal kann eine Gleichspannung, ein Gleichfeld, eine niederfrequente Wechselspannung oder ein niederfrequentes Wechselfeld sein.

In einigen Ausführungsformen weist das erste Anregungssignal eine Leistung in einem Bereich von 1 bis 200 mW, insbesondere von 50 bis 100 mW auf.

In einigen Ausführungsformen ist das zweite Anregungssignal ein elektrisches Gleichspannungsfeld von 10 bis 50 V, insbesondere 20 bis 40 V.

In einigen Ausführungsformen ist das zweite Anregungssignal ein Gleichsignal, das größer ist als die Amplitude des ersten Anregungssignals. Beispielsweise ist das zweite Anregungssignal ein Gleichstrom oder eine Gleichspannung und das erste Anregungssignal ist ein Wechselstrom oder eine Wechselspannung, wobei insbesondere der Gleichstrom bzw. die Gleichspannung größer als die Amplitude des Wechselstroms bzw. der Wechselspannung ist.

In einigen Ausführungsformen ist das Löschsignal ein abklingendes Wechselfeld mit einer abnehmenden Leistung beginnend bei 2 W oder weniger und/oder beginnend bei 1 W oder mehr.

In einigen Ausführungsformen umfasst die Erfassungsvorrichtung einen Temperatursensor für die Blut- und/oder Körpertemperatur. In einigen Ausführungsformen umfasst das Verfahren das Messen der Blut- und/oder Körpertemperatur, insbesondere der Körperkerntemperatur und/oder der Hautoberflächentemperatur.

In einigen Ausführungsformen misst das Verfahren und/oder die Erfassungsvorrichtung den Blutbildparameter am Handgelenk, am Finger und/oder an der Zunge.

Die gewählten Frequenzen und Frequenzbereiche, Spannungen, Stromstärken und Leistungen können in einigen Fällen einige oder alle der folgende Vorteile bieten: Abhängig von den gewählten Frequenzbereichen, Spannungen, Stromstärken und Leistungen können Ankopplungsstrukturen, wie beispielsweise Antennen, der Erfassungsvorrichtung in besonders vorteilhaften Abmessungen gestaltet werden. Abhängig von den gewählten Frequenzbereichen, Spannungen, Stromstärken und Leistungen kann eine besonders energieeffiziente Messung durchgeführt werden. Die Frequenzbereiche können Resonanzfrequenzen von Wasser in Blut bei Körpertemperatur und bei unterschiedlichen Konzentrationen eines Blutbildparameters enthalten, wodurch der Blutbildparameter besonders genau gemessen werden kann. Die Frequenzbereiche, Spannungen, Stromstärken und Leistungen können eine vorteilhafte Eindringtiefe der Anregungssignale erlauben, so dass besonders relevante Messdaten erzeugt werden können.

## BESCHREIBUNG DER FIGUREN

Weitere Ausführungsbeispiele werden bezugnehmend auf die beiliegenden Figuren näher erläutert:

Fig. 1 zeigt eine schematische Darstellung einer Erfassungsvorrichtung zum Erfassen eines Blutbildparameters in einem Blutgefäß gemäß einer Ausführungsform;

Fig. 2 zeigt eine schematische Darstellung einer Erfassungsvorrichtung zum Erfassen eines Blutbildparameters in einem Blutgefäß gemäß einer Ausführungsform;

Fig. 3a zeigt eine schematische Darstellung einer Reflexions- / Transmissionsmessung zur Erfassung einer Änderung des elektromagnetischen Feldes in einem Blutgefäß mittels einer Erfassungsvorrichtung gemäß einer Ausführungsform;

Fig. 3b zeigt eine schematische Darstellung einer Reflexions- / Transmissionsmessung zur Erfassung einer Änderung des elektromagnetischen Feldes in einem Blutgefäß mittels einer Erfassungsvorrichtung gemäß einer Ausführungsform;

Fig. 4 zeigt eine S-Parameter Darstellung einer Kopplung einer Antennenanordnung bzw. Leitungsanordnung einer Erfassungsvorrichtung mit einem Blutgefäß gemäß einer Ausführungsform;

Fig. 5 zeigt ein Diagramm zur Illustrierung einer reellen Dielektrizitätszahl $\varepsilon'$ und einer komplexen Dielektrizitätszahl $\varepsilon''$ in Abhängigkeit von einer Frequenz, welche von der Erfassungsvorrichtung bestimmt wird, gemäß einer Ausführungsform;

Fig. 6 zeigt eine schematische Darstellung eines Verfahrens zum Erfassen eines Blutbildparameters in einem Blutgefäß gemäß einer Ausführungsform; und

Fig. 7 zeigt eine schematische Darstellung eines Diagramms zum Auslöschen einer Polarisation von Dipolen in einem Blutgefäß, welche durch ein von einer Erfassungsvorrichtung erzeugtes Kalibrierungssignal gemäß einer Ausführungsform verursacht ist.

## DETAILLIERTE BESCHREIBUNG DER FIGUREN

In der folgenden ausführlichen Beschreibung wird auf die beiliegenden Zeichnungen Bezug genommen, die einen Teil hiervon bilden und in denen als Veranschaulichung spezifische Ausführungsformen gezeigt sind, in denen die Erfindung ausgeführt werden kann. Es versteht sich, dass auch andere Ausführungsformen genutzt und strukturelle oder logische Änderungen vorgenommen werden können, ohne von dem Konzept der vorliegenden Erfindung abzuweichen. Die folgende ausführliche Beschreibung ist deshalb nicht in einem beschränkenden Sinne zu verstehen. Ferner versteht es sich, dass die Merkmale der verschiedenen hierin beschriebenen Ausführungsbeispiele miteinander kombiniert werden können, sofern nicht spezifisch etwas anderes angegeben ist.

Die Aspekte und Ausführungsformen werden unter Bezugnahme auf die Zeichnungen beschrieben, wobei gleiche Bezugszeichen sich im Allgemeinen auf gleiche Elemente beziehen. In der folgenden Beschreibung werden zu Erläuterungszwecken zahlreiche spezifische Details dargelegt, um ein eingehendes Verständnis von einem oder mehreren Aspekten der Erfindung zu vermitteln. Für einen Fachmann kann es jedoch offensichtlich sein, dass ein oder mehrere Aspekte oder Ausführungsformen mit einem geringeren Grad der spezifischen Details ausgeführt werden können. In anderen Fällen werden bekannte Strukturen und Elemente in schematischer Form dargestellt, um das Beschreiben von einem oder mehreren Aspekten oder Ausführungsformen zu erleichtern. Es versteht sich, dass andere Ausführungsformen genutzt und strukturelle oder logische Änderungen vorgenommen werden können, ohne von dem Konzept der vorliegenden Erfindung abzuweichen.

Wenngleich ein bestimmtes Merkmal oder ein bestimmter Aspekt einer Ausführungsform bezüglich nur einer von mehreren Implementierungen offenbart worden sein mag, kann außerdem ein derartiges Merkmal oder ein derartiger Aspekt mit einem oder mehreren anderen Merkmalen oder Aspekten der anderen Implementierungen kombiniert werden, wie für eine gegebene oder bestimmte Anwendung erwünscht und vorteilhaft sein kann. Weiterhin sollen in dem Ausmaß, in dem die Ausdrücke "enthalten", "haben", "mit" oder andere Varianten davon entweder in der ausführlichen Beschreibung oder den Ansprüchen verwendet werden, solche Ausdrücke auf eine Weise ähnlich dem Ausdruck "umfassen" einschließend sein. Die Ausdrücke "gekoppelt" und "verbunden" können zusammen mit Ableitungen davon verwendet worden sein. Es versteht sich, dass derartige Ausdrücke dazu verwendet werden, um anzugeben, dass zwei Elemente unabhängig davon miteinander kooperieren oder interagieren, ob sie in direktem physischem oder elektrischem Kontakt stehen oder nicht in direktem Kontakt miteinander stehen. Außerdem ist der Ausdruck "beispielhaft" lediglich als ein Beispiel aufzufassen anstatt der Bezeichnung für das Beste oder Optimale. Die folgende Beschreibung ist deshalb nicht in einem einschränkenden Sinne zu verstehen.

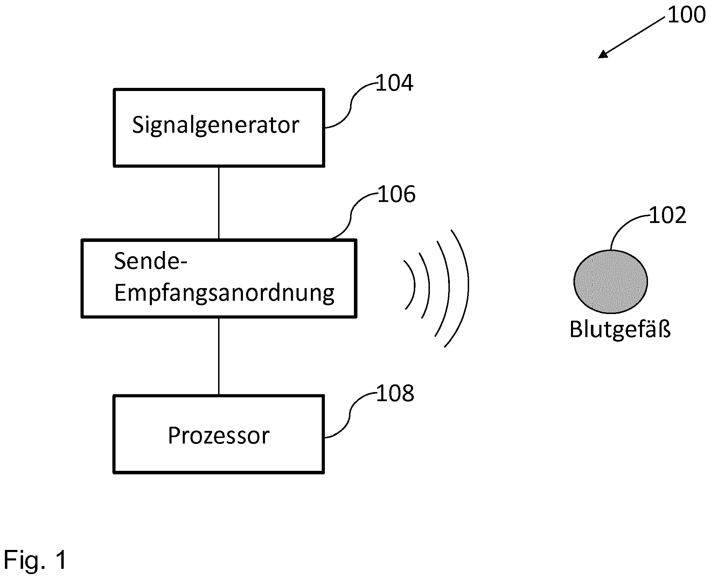

Fig. 1 zeigt eine schematische Darstellung einer Erfassungsvorrichtung 100 zum Erfassen eines Blutbildparameters in einem Blutgefäß 102 gemäß einer Ausführungsform.

Die Erfassungsvorrichtung 100 umfasst einen Signalgenerator 104, welcher ausgebildet ist, ein Kalibrierungsmesssignal zu erzeugen, wobei das Kalibrierungsmesssignal eine Überlagerung aus einem ersten elektrischen Anregungssignal und einem zweiten Anregungssignal umfasst, wobei das erste elektrische Anregungssignal eine höhere Frequenz als das zweite Anregungssignal aufweist, und wobei das zweite Anregungssignal eine höhere elektrische Leistung als das erste elektrische Anregungssignal aufweist.

Weiterhin umfasst die Erfassungsvorrichtung 100 eine Sende-Empfangsanordnung 106, welche ausgebildet ist, das Kalibrierungsmesssignal in Richtung des Blutgefäßes 102 auszusenden und ansprechend auf das Aussenden des Kalibrierungssignals ein erstes Systemantwortsignal zu empfangen, wobei die Sende-Empfangsanordnung 106 ausgebildet ist, ein drittes Anregungssignal in Richtung des Blutgefäßes 102 auszusenden und ansprechend auf das Aussenden des dritten Anregungssignals ein zweites Systemantwortsignal zu empfangen.

Ferner umfasst die Erfassungsvorrichtung 100 einen Prozessor 108, welcher ausgebildet ist, das erste Systemantwortsignal und das zweite Systemantwortsignal zu verknüpfen, um ein Messsignal zur Bestimmung des Blutbildparameters zu erhalten.

Der Signalgenerator 104 kann ausgebildet sein, das erste elektrische Anregungssignal als ein Kleinsignal und das zweite Anregungssignal als ein Großsignal zu erzeugen. Das Großsignalverhalten eines Untersuchungsmaterials ("Material Under Test", MUT), z. B. des Blutes, ist nicht identisch zum Kleinsignalverhalten. Dies geschieht dadurch, dass dem Kleinsignal, z. B. einem Mikrowellensignal, das Großsignal überlagert werden kann. Dieses Großsignal könnte z. B. eine Gleichspannung bzw. ein Gleichfeld oder eine niederfrequente Wechselspannung bzw. ein niederfrequentes Wechselfeld sein.

Durch die Überlagerung des Großsignals und des Kleinsignals wird der Vorteil erreicht, dass das Kalibrierungssignal oder eine Referenzkurve bestimmt werden können, so dass alle Unzulänglichkeiten und Variationen der analogen Messschaltung als auch der hochfrequenten (HF) Leitungen beinhaltet sind. Daher kompensiert eine Kalibrierung mittels des Kalibrierungssignals beispielsweise Variationen in der Analogschaltungstechnik.

Weiterhin wird durch die Erfassungsvorrichtung 100 gemäß dem ersten Aspekt beispielsweise der technische Vorteil erreicht, dass ein Kalibrierungsmesssignal erzeugt werden kann, welches keine Referenzflüssigkeiten benötigt, welches kurz vor der eigentlichen Glukosemessung in dem Blut erzeugt werden kann, und welches keine große Interaktion durch den Patienten erfordert. Weiterhin wird dadurch der Vorteil erreicht, dass das Messsignal zur Bestimmung des Blutbildparameters nicht-invasiv erhalten werden kann.

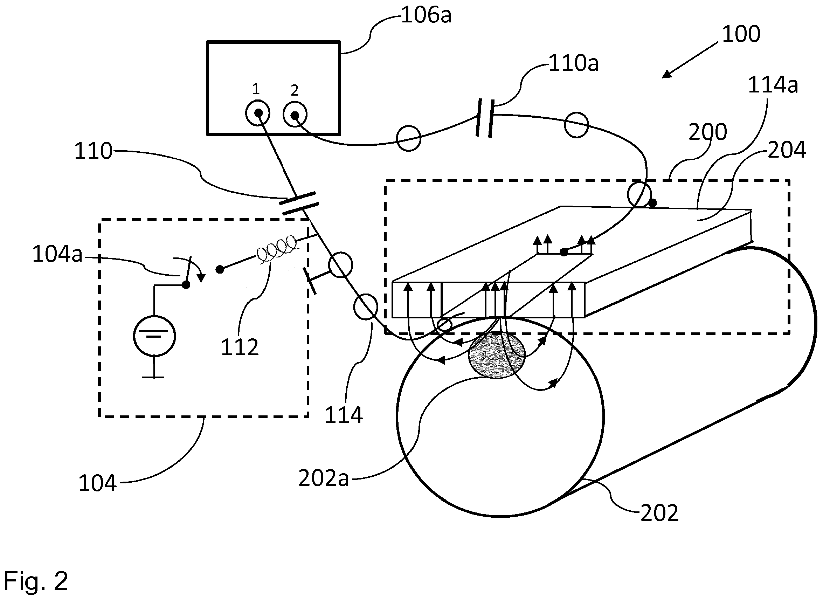

Fig. 2 zeigt eine schematische Darstellung einer Erfassungsvorrichtung 100 zum Erfassen eines Blutbildparameters in einem Blutgefäß 102 gemäß einer Ausführungsform.

In dieser Ausführungsform der Erfassungsvorrichtung 100 umfasst der Signalgenerator 104 ein Bias-T 112, welches ausgebildet ist, ein Großsignal von mehreren Volt einem schwachen Kleinsignal, z. B. einem Mikrowellensignal im mV-Bereich, zu überlagern. Der Signalgenerator 104 kann ferner einen Schalter 104a umfassen, wobei der Schalter 104a ausgebildet ist, die Überlagerung des Großsignals ein- oder auszuschalten. Ferner umfasst die Erfassungsvorrichtung 100 zwei Kondensatoren 110 und 110a, welche in einem Wechselstromkreis der Erfassungsvorrichtung 100 als Wechselstromwiderstand mit einem frequenzabhängigen Impedanzwert wirken.

Der Schalter 104a kann ferner ausgebildet sein, ein Wechselsignal (AC) aufzuschalten.

Ferner kann die Erfassungsvorrichtung 100 eine Antennenanordnung (Ankoppelanordnung) 200 umfassen, welche an einem Arm 202 des menschlichen Körpers befestigt sein kann, und welche ausgebildet sein kann, die elektromagnetische Energie der Anregungssignale in einer Ader 202a zu fokussieren. In dieser Ausführungsform umfasst die Antennenanordnung 200 eine elektrische Leitungsanordnung 204, welche als Mikrostreifenleitung ausgestaltet sein kann, so dass ein Leitungsstreifen als Erreger zur Einkopplung von Mikrowellenenergie und ein anderer Leitungsstreifen als Empfänger zum Empfangen der Mikrowellenenergie dient. Die Antennenanordnung 200 kann eine durch die Ader 202a dielektrisch belastete Mikrostreifenleitung mit zwei elektrischen Toren 114, 114a umfassen. Die Sende-Empfangsanordnung 106 kann ferner einen Netzwerkanalysator (NWA) 106a umfassen, welcher mit den zwei elektrischen Toren 114, 114a verbunden ist, und welcher ausgebildet ist, die Streuparameter (S-Parameter), d. h. die Wellengröße der Reflexion (insbesondere einen Vorwärts-Reflexionsfaktor $S_{11}$) und der Transmission (insbesondere einen Vorwärts-Transmissionsfaktor $S_{21}$), an elektrischen Toren 114, 114a als Funktion der Frequenz zu messen.

Gemäß einer Ausführungsform weist die elektrische Leitungsanordnung 204 eine Länge im Bereich von etwa 1 mm bis etwa 20 mm auf. Referenzmessungen haben gezeigt, dass sich der Blutbildparameter umso genauer bestimmen lässt, je länger die Leitung ist. Bei längeren Leitungen kann mehr Mikrowellenenergie in das Blutgefäß eingekoppelt werden, wodurch die Änderung des elektromagnetischen Feldes aufgrund der Belastung durch das Blutgefäß 102 stärker in Ausprägung tritt. Allerdings sollte ein Kompromiss zwischen Leitungslänge und Handhabbarkeit der elektrischen Leitungsanordnung 204 geschlossen werden. Wenn beispielsweise die elektrische Leitungsanordnung 204 mittels einer Anpressmanschette auf die Haut gepresst werden soll, ist der Platz für die elektrische Leitungsanordnung 204 begrenzt. Ferner verlaufen die Blutgefäße 102 nicht immer geradlinig durch den Körper, so dass bei größeren Längen nicht die gesamte Leitungslänge auf das Blutgefäß 102 einwirken kann. Eine Länge in einem Bereich von etwa 1 mm bis etwa 20 mm oder 30 mm hat sich bezüglich Handhabbarkeit als vorteilhaft erwiesen. Dadurch wird auch eine gute Integrierbarkeit in einem Armband ermöglicht.

Gemäß einer Ausführungsform besteht die Mikrostreifenleitung aus einem leitfähigen Streifen, der durch ein dielektrisches Substrat von einer leitfähigen Fläche getrennt ist. Die Mikrostreifenleitung kann aus einem nicht leitenden Substrat bzw. einer Leiterplatte bestehen, welche auf der Unterseite vollständig metallisiert ist, so dass die Metallisierung als Massefläche dient. Auf der Oberseite kann ein Leiter in Form eines Streifens bzw. einer Leiterbahn, also mit definierter Leiterbahnbreite und Leiterbahnlänge, angeordnet sein. Dieser Streifen kann gewöhnlich durch Bearbeitung der oberen Metallisierung durch Ätzen oder Fräsen angefertigt werden. Als Substrat können verschiedene Dielektrika, beispielsweise glasfaserverstärktes PTFE (Polytetrafluorethylen), Aluminiumoxid oder ein anderes keramisches Material, verwendet werden. Das von der elektrischen Leitungsanordnung 204 abgestrahlte Signal breitet sich zum einen in dem Zwischenraum zwischen dem Streifenleiter und der Massefläche aus, zum anderen treten die Feldlinien auch in den freien Raum über dem Streifenleiter ein, der in der Regel mit Luft gefüllt ist, hier aber durch das Blutgefäß und seine Umgebung gebildet wird. Ein Hohlleiter kann über dem Streifenleiter angeordnet sein, um die elektrische Leitungsanordnung 204 anzuregen.

Wie bereits oben erwähnt kann die Mikrostreifenleitung aus einem nicht leitenden Substrat bestehen, welches auf der Unterseite metallisiert ist und welches auf der Oberseite einen Leiter in Form eines Streifens aufweist. Die durch die Sende-Empfangsanordnung 106 gesendete Mikrowellenenergie breitet sich zum einen in dem Zwischenraum zwischen dem Streifenleiter und der Metallisierung aus, zum anderen treten Feldlinien auch in den freien Raum über dem Streifenleiter ein. Legt man den Streifenleiter an die Haut an, so kann die Mikrowellenenergie mittels der Feldlinien aus dem Streifenleiter austreten, in das Blutgefäß 102 eintreten und wieder zurück in die elektrische Leitungsanordnung 204 einkoppeln. Wie bereits oben erwähnt, bildet das Blutgefäß 102 einen Widerstand für die Mikrowellenenergie, welche die elektrische Leitungsanordnung 204 belastet und damit die Anordnung der Feldlinien beeinflusst. Statt einer Mikrowellenleitung kann auch ein geschlitzter Hohlleiter verwendet werden, der denselben Effekt bewirkt, wie oben beschrieben. Während Mikrostreifenleitungen sich insbesondere für den Einsatz im Frequenzbereich zwischen einigen hundert Megahertz und etwa 20 Gigahertz eignen, eignen sich Hohlleiter insbesondere für den Einsatz im Zentimeter-Wellenbereich und darunter, d. h. von etwa 3 GHz bis etwa 200 GHz. Bei Frequenzen bis etwa 20 GHz lässt sich somit bevorzugt die Mikrostreifenleitung einsetzen, welche einfach und handlich zu fertigen ist, während für den Einsatz von Frequenzen über etwa 20 GHz bevorzugt eine Hohlleiterstruktur zum Einsatz kommen kann, welche eine sehr präzise Messung erlaubt.

Gemäß einer Ausführungsform umfasst der geschlitzte Hohlleiter einen Rechteckhohlleiter mit einer Mehrzahl kreisförmiger Schlitze oder mit einem rechteckförmigen Schlitz, insbesondere mit einem rechteckförmigen Schlitz der Breite 1 mm und der Länge 20 mm. Rechteckhohlleiter eignen sich insbesondere für den Einsatz im Zentimeter-Wellenbereich und darunter, d. h. von etwa 3 GHz bis etwa 200 GHz, und gestatten damit eine sehr präzise Erfassung des Blutbildparameters. Wie bereits oben erläutert, haben Referenzmessungen gezeigt, dass sich der Blutbildparameter umso genauer bestimmen lässt, je länger die Leitung ist. Bei geschlitzten Hohlleitern gelten diese Aussagen entsprechend für die Länge des Schlitzes, durch welchen die Mikrowellenenergie ausgekoppelt wird. Bei längeren Schlitzen kann mehr Mikrowellenenergie in das Blutgefäß 102 eingekoppelt werden, wodurch die Änderung des elektromagnetischen Feldes aufgrund der Belastung durch das Blutgefäß stärker in Ausprägung tritt. Allerdings sollte ein Kompromiss zwischen Schlitzlänge und Handhabbarkeit der elektrischen Leitungsanordnung 204 geschlossen werden. Da Hohlleiter sehr sperrig sein können, sollte die Schlitzlänge möglichst begrenzt werden. Eine Länge in einem Bereich von etwa 1 mm bis etwa 20 oder 30 mm hat sich bezüglich seiner Handhabbarkeit als vorteilhaft erwiesen.

Gemäß einer Ausführungsform umfasst die Mikrostreifenleitung eine koplanare Mikrostreifenleitung, insbesondere eine masselose koplanare Mikrostreifenleitung mit einer Spaltbreite im Bereich von etwa 0,1 mm bis etwa 0,9 mm. Referenzmessungen haben gezeigt, dass der Effekt der Feldänderung in diesem Spaltbreitenbereich besonders ausgeprägt ist.

Gemäß einer Ausführungsform kann die Kopplung der elektrischen Leitungsanordnung 204 an das Blutgefäß durch nichtinvasives Befestigen der elektrischen Leitungsanordnung 204 an einer Hautoberfläche eines menschlichen oder tierischen Körpers erfolgen.

Das Befestigen an der Hautoberfläche kann ein reines Auflegen auf die Haut sein, beispielsweise bei Verwendung eines dünnen Mikrostreifenleiters, der sich an die Unebenheiten der Hautoberfläche anschmiegt. Vorzugsweise wird zum Befestigen aber eine Befestigungseinrichtung verwendet, um für einen geeigneten Anpressdruck zu sorgen, damit die Messungen die erforderliche Genauigkeit aufweisen. Beispielsweise kann eine Anpressmanschette verwendet werden, wie sie bei Blutdruckmessgeräten verwendet wird, um einen Luftspalt zu vermeiden. Gemäß einer Ausgestaltung der Ausführungsform umfasst die Erfassungsvorrichtung 100 ein an einem Arm 202 befestigbares Armband.

Die Erfassungsvorrichtung 100 kann auch als eine in-situ Erfassungsvorrichtung bezeichnet werden, da die Erfassungsvorrichtung 100 sowohl für die Bestimmung des Kalibrierungssignals oder der Referenzkurve als auch für die Bestimmung des Messsignals selbst auf dem Körper verbleiben kann. Es sind keine Referenzflüssigkeiten notwendig.

Zwischen der Referenzmessung und der eigentlichen Messung vergeht kaum Zeit (Größenordnung Sekunde). Damit kann eine Drift der Analogschaltung zwischen der Referenzmessung und eigentlicher Messung ausgeschlossen werden.

Ein weiterer Vorteil ist zudem, dass auch davon ausgegangen werden kann, dass die Erfassungsvorrichtung 100 zwischen der Referenzmessung und der eigentlichen Messung nicht verrutscht. Die Antennenanordnung 200 sieht die identische dielektrische Umgebung. Daher kann die Erfassungsvorrichtung 100 mittels des Großsignals gleichzeitig Variationen in der Glukosemessung bedingt durch eine Drift der Analogschaltungstechnik wie auch durch eine nichtreproduzierbare Ankopplung an den Körper eliminieren.

Gerade auch die Variation in der elektromagnetischen Ankopplung an den Körper, da z. B. das Armband immer wieder verrutscht, stellt ein großes Problem dar. Dieses wird ebenfalls durch die Erfassungsvorrichtung 100 gelöst. Hierzu ist lediglich über das Bias-T 112 dem Mikrowellensignal zur Messung der Streuparameter (typischerweise 15,...,25 GHz) ein Großsignal in Form eines DC Signals oder eines niederfrequenten AC Signals zu überlagern. Das Großsignal ist dann nur ein- und auszuschalten, um zwischen Messung der Referenzkurve und eigentlicher Messung umzuschalten.

Durch das überlagerte Großsignal kann das Kalibrierungssignal oder eine Referenzkurve bestimmt werden, in welcher alle Unzulänglichkeiten und Variationen der analogen Messschaltung als auch der hochfrequenten (HF) Leitungen beinhaltet sind. Dadurch wird der Vorteil erreicht, dass die Antennenanordnung 200 an den Körper in gleicher Weise für das Klein- wie Großsignal verwendet werden kann. Damit kompensiert die Kalibrierung sowohl Variationen in der Analogschaltungstechnik als auch in der Ankopplungstechnik.

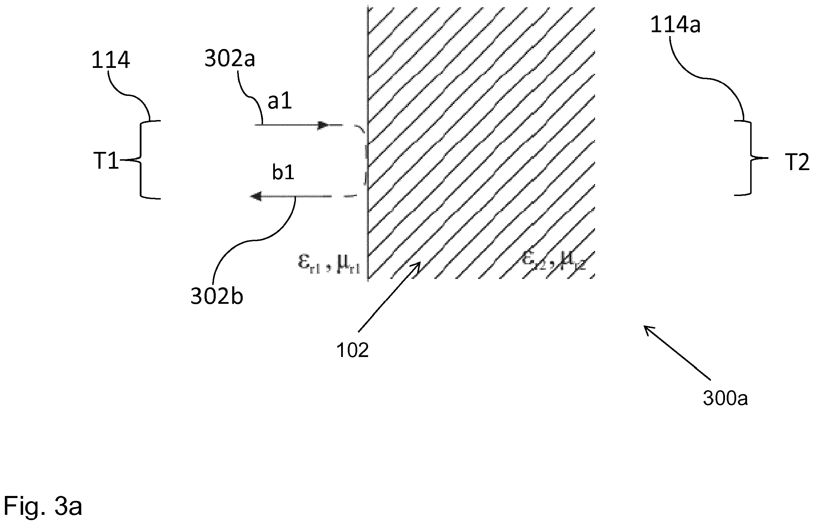

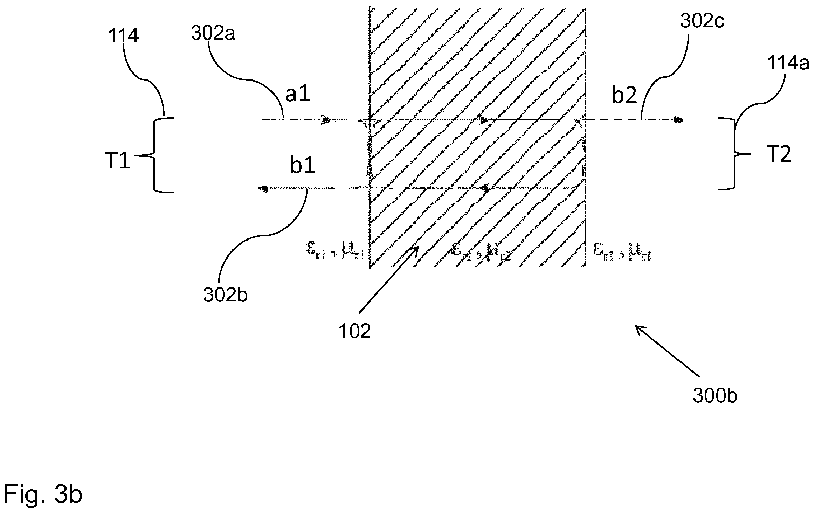

Fig. 3a und Fig. 3b zeigen eine schematische Darstellung einer Reflexions- 300a / Transmissionsmessung 300b zur Erfassung einer Änderung des elektromagnetischen Feldes in einem Blutgefäß 102 gemäß einer Ausführungsform.

Das Blutgefäß 102 lässt sich durch eine komplexe Dielektrizitätszahl $\varepsilon$ charakterisieren, wobei der Realteil $\varepsilon'$ der komplexen Dielektrizitätszahl $\varepsilon$ im Wesentlichen die Polarisierbarkeit eines Stoffes im Blut abbildet,

und der Imaginärteil $\varepsilon''$ dessen Verluste abbildet. Insbesondere beschreibt der Realteil $\varepsilon'$ der komplexen Dielektrizitätszahl $\varepsilon$ die dielektrische Leitfähigkeit, auch Permittivität oder $\varepsilon_{r2}$ genannt, des Blutgefäßes 102, da die Dielektrizitätszahl $\varepsilon$ als das dimensionslose Verhältnis der Permittivität des Materiales zur Permittivität des Vakuums definiert ist.

Neben seiner dielektrischen Leitfähigkeit $\varepsilon_{r2}$ lässt sich das Blutgefäß 102 auch durch seine magnetische Leitfähigkeit $\mu_{r2}$ charakterisieren. Diese Parameter unterscheiden sich von der dielektrischen Leitfähigkeit $\varepsilon_{r1}$ und der magnetischen Leitfähigkeit $\mu_{r1}$ außerhalb des Blutgefäßes 102. Weiterhin kann das Blutgefäß 102 als ein Zweitor modelliert werden und mittels Streuparametermessungen vermessen werden.

Bei der Reflexionsmessung 300a wird an dem ersten Tor T1 114 eine einfallende Welle $a_1$ 302a in Richtung des Blutgefäßes 102 abgestrahlt. Bei Einfall einer elektromagnetischen Welle $a_1$ 302a auf die Wand des Blutgefäßes 102 wird die Welle aufgrund der Unterschiede in den elektrischen und magnetischen Leitfähigkeiten von dem Blutgefäß 102 reflektiert und kann als reflektierte Welle b1 302b am Tor T1 114 gemessen werden. Am Tor T2 114a findet keine Aktivität statt.

Bei der Transmissionsmessung 300b wird an dem ersten Tor T1 114 die einfallende Welle $a_1$ 302a in Richtung des Blutgefäßes 102 abgestrahlt. Bei Einfall einer elektromagnetischen Welle $a_1$ 302a auf die Wand des Blutgefäßes 102 wird die Welle aufgrund der Unterschiede in den elektrischen und magnetischen Leitfähigkeiten teilweise von dem Blutgefäß 102 reflektiert. Ein Teil der Energie wird als reflektierte Welle $b_1$ 302b am Tor T1 reflektiert und kann dort gemessen werden. Ein weiterer Teil der Energie durchdringt das Blutgefäß 102 und kann als übertragene Welle $b_2$ 302c am Tor T2 114a gemessen werden.

Bei der Modellierung des Blutgefäßes 102 mittels Zweitordarstellung umfassen die S-Parameter die Elemente $S_{11}$, $S_{12}$, $S_{21}$ und $S_{22}$:

$$\begin{pmatrix} b_1 \\ b_2 \end{pmatrix} = \begin{pmatrix} S_{11} & S_{12} \\ S_{21} & S_{22} \end{pmatrix} \begin{pmatrix} a_1 \\ a_2 \end{pmatrix}.$$

Dabei entspricht: $a_1$ der am Tor T1 114 einlaufenden Welle 302a, $a_2$ der am Tor T2 114a einlaufenden Welle, $b_1$ der vom Eingang (Tor T1, 114) auslaufenden Welle, und $b_2$ der vom Ausgang (Tor T2, 114a) auslaufenden Welle 302c.

Die Bedeutung der Elemente $S_{11}$, $S_{12}$, $S_{21}$ und $S_{22}$ der S-Parameter wird im Folgenden näher erläutert.

Der Eingangs-Reflexionsfaktor $S_{11}$:

$$S_{11} = \left. \frac{b_1}{a_1} \right|_{a_2=0}$$

stellt die Reflexion am Eingang ohne Welleneinprägung am Tor T1 114 dar.

Der Ausgangs-Reflexionsfaktor $S_{22}$:

$$S_{22} = \left.\frac{b_2}{a_2}\right|_{a_1=0}$$

stellt die Reflexion am Tor T2 114a ohne Anregung an Tor T1 114 dar.

Der Vorwärts-Transmissionsfaktor $S_{21}$:

$$S_{21} = \left.\frac{b_2}{a_1}\right|_{a_2=0}$$

stellt die Vorwärts-Transmission ohne Anregung am Tor T2 114a dar.

Der Rückwärts-Transmissionsfaktor $S_{12}$:

$$S_{12} = \left.\frac{b_1}{a_2}\right|_{a_1=0}$$

stellt die Rückwärts-Transmission ohne Anregung am Tor T1 114 dar.

Zur Messung von $|S_{21}|$ kann der in Fig. 2 dargestellte Netzwerkanalysator 106a einen durchstimmbaren Oszillator umfassen.

Gemäß einem weiteren Ausführungsbeispiel kann durch eine weitere Messung eines Betrags des Messparameters von $S_{11}$ die Genauigkeit bei der Bestimmung der Verlustgrößen noch weiter gesteigert werden. Die Verlustgrößen können beispielsweise auf der Basis der folgenden Formel bestimmt werden:

$$P_{\text{Verlust}} = 1 - |S_{11}|^2 - |S_{21}|^2,$$

wobei $P_{\text{Verlust}}$ die jeweilige Verlustgröße bezeichnet, und wobei $S_{11}$ den Eingangs-Reflexionsfaktor und $S_{21}$ den Vorwärts-Transmissionsfaktor bezeichnen.

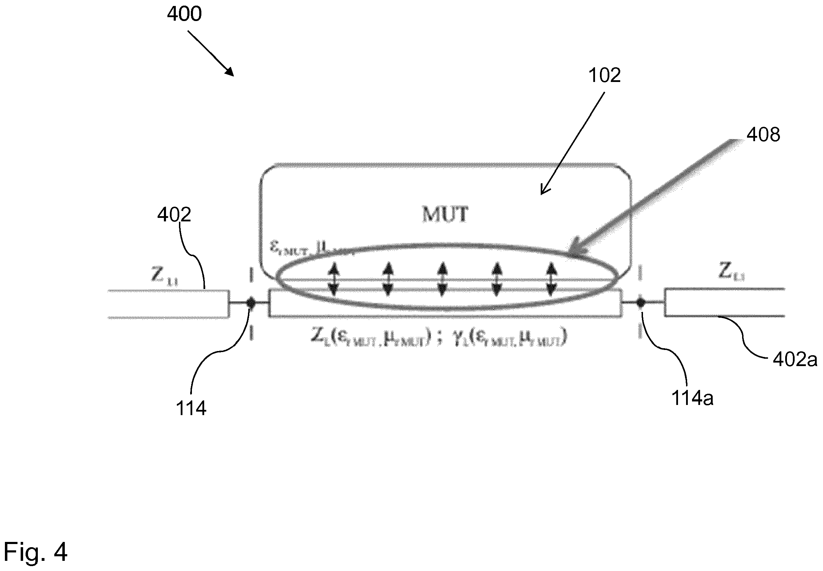

Fig. 4 zeigt eine S-Parameter Darstellung 400 der Kopplung der elektrischen Leitungsanordnung 204 mit dem Blutgefäß 102 gemäß einer Ausführungsform.

Dabei wird die elektrische Leitungsanordnung 204 an den beiden Toren T1 114 und T2 114a mit Mikrowellenenergie angeregt. Die Sende-Empfangsanordnung 106 kann ausgebildet sein, die elektrische Leitungsanordnung 204 anzuregen und die Änderung des elektromagnetischen Feldes durch die Tore T1 114 und T2 114a zu erfassen.

Die beiden Impedanzen $Z_{L_1}$ links 402 und rechts 402a der beiden Tore T1 114 und T2 114a stellen beispielsweise Innenwiderstände der Sende-Empfangsanordnung 106 oder der Erfassungsvorrichtung 100 dar, während die Impedanz $Z_L(\epsilon_{r,MUT}, \mu_{r,MUT})$ zwischen den beiden Toren T1 114 und T2 114a den Wellenwiderstand der mit dem Blutgefäß 102 belasteten elektrischen Leitungsanordnung 204 bezeichnet. Das Blutgefäß 102 wird hier auch durch die Bezeichnung "MUT, material under test" spezifiziert. Bei der Ausbreitung von elektromagnetischen Wellen 408 auf der elektrischen Leitungsanordnung 204 entsteht am Leitungsende eine Reflexion, wenn die dort angeschlossene Schaltung eine vom Wert der Wellenimpedanz $Z_L(\epsilon_{r1}, \mu_{r1})$ der unbelasteten elektrischen Leitungsanordnung 204 abweichende Eingangsimpedanz $Z_L(\epsilon_{r2}, \mu_{r2})$ besitzt. Das Verhältnis von reflektierter zu hinlaufender Spannungswelle wird als Reflexionsfaktor bezeichnet und wird nach folgender Gleichung berechnet:

$$r = \frac{\vec{E}_{2tan}^-}{\vec{E}_{1tan}^+} = \frac{Z_2(\epsilon_{r2}, \mu_{r2}) - Z_1(\epsilon_{r1}, \mu_{r1})}{Z_2(\epsilon_{r2}, \mu_{r2}) + Z_1(\epsilon_{r1}, \mu_{r1})},$$

dabei entsprechen:

$Z_L(\epsilon_{r1}, \mu_{r1})$: dem Wellenwiderstand der unbelasteten elektrischen Leitungsanordnung 204;
$Z_L(\epsilon_{r2}, \mu_{r2})$: dem Eingangswiderstand des an die elektrische Leitungsanordnung 204 gekoppelten Blutgefäßes 102;
$E_{1tan}$: der Feldstärke der hinlaufenden Welle 302a; und
$E_{2tan}$: der Feldstärke der rücklaufenden Welle 302b.

Für $Z_L(\epsilon_{r1}, \mu_{r1}) = Z_L(\epsilon_{r2}, \mu_{r2})$ wird der Reflexionsfaktor zu Null. Dieser Zustand wird angestrebt, da dann die maximale Leistung übertragen wird und die Messwertauflösung am größten ist, um den Blutbildparameter zu bestimmen. Man spricht dann von Impedanzanpassung oder Leistungsanpassung.

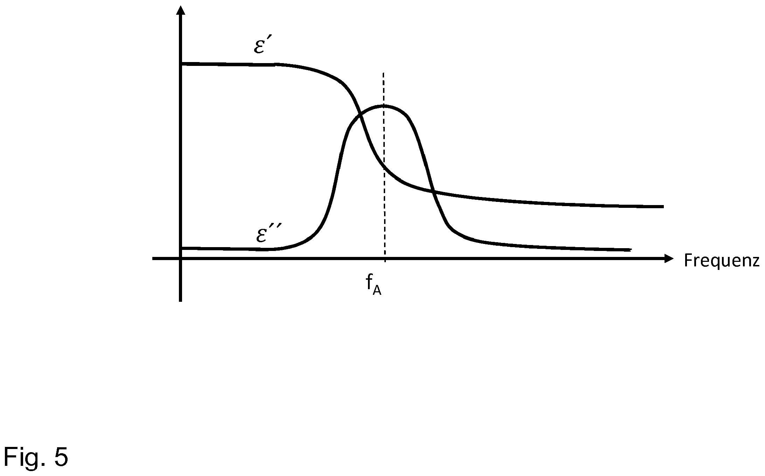

Fig. 5 zeigt ein Diagramm zur Illustrierung einer reellen Dielektrizitätszahl ε' und einer komplexen Dielektrizitätszahl ε'' in Abhängigkeit der Frequenz, welche von der Erfassungsvorrichtung 100 bestimmt werden, gemäß einer Ausführungsform.

Gemäß einer Ausführungsform ist der Prozessor 108 oder die Sende-Empfangsanordnung 106 ausgebildet, zur Bestimmung des Blutbildparameters eine komplexe Dielektrizitätszahl ε zu bestimmen, wobei der Realteil ε' der komplexen Dielektrizitätszahl ε im Wesentlichen die Polarisierbarkeit eines Stoffes im Blut abbildet, und der Imaginärteil ε'' dessen Verluste abbildet.

Üblicherweise werden Relaxationsphänomene in der dielektrischen Spektroskopie, auf welcher die Erfassungsvorrichtung 100 basiert, durch Cole-Cole Relaxationen beschrieben. Eine Cole-Cole Relaxationskurve beschreibt die komplexe Dielektrizitätskonstante:

$$\varepsilon = \varepsilon' + j\varepsilon''$$

als Funktion der Frequenz. Die Cole-Cole Relaxation stellt eine Überlagerung vieler Debye Relaxationen dar.

Wie oben beschrieben, bildet der Realteil $\varepsilon'$ der komplexen Dielektrizitätszahl $\varepsilon$ im Wesentlichen die Polarisierbarkeit eines Stoffes und der Imaginärteil $\varepsilon''$ seine Verluste ab. Eine Relaxationsfrequenz geht einher mit einem Maximum des Imaginärteils $\varepsilon''$. Gleichzeitig fällt der Realteil $\varepsilon'$ bei der Relaxationsfrequenz $f_A$ um eine Stufe ab. Das Verhalten der komplexen Dielektrizitätskonstante oder Dielektrizitätszahl $\varepsilon$ geht dann einher mit einem Maximum der Transmissionsdämpfung der Streuparameter. Findet man also in der Streuparametermessung eine Frequenz, bei der hohe Verluste auftreten, hat man eine Relaxationsfrequenz $f_A$ gefunden, da hier der Imaginärteil $\varepsilon''$ maximal ist. Dieser Anstieg der Verluste wird in der Spektroskopie auch als Relaxationsmechanismus bezeichnet. Der Effekt, der hierbei ausgenutzt werden kann, ist der, dass sich die Frequenz, bei der die Überhöhung der Verluste - siehe lokales Maximum von $\varepsilon''$ - auftritt, mit der Konzentration des Zuckergehaltes verschiebt.

Beispielsweise besteht der menschliche Körper zu 80% aus Wasser. Das Wasser besitzt einen Relaxationsmechanismus z. B. bei ca. 20 GHz. Deren Verstimmung kann bestimmt werden und auf den Zuckergehalt abgebildet werden. Die Verstimmung der Resonanzfrequenz $f_A$ bei $\varepsilon''$ ist leichter detektierbar als die Plateauveränderung von $\varepsilon'$. Insbesondere verschieben Variationen in der Ankopplung die Frequenz des Maximums von $\varepsilon''$ vorteilhafterweise nicht. Damit ist eine Bestimmung der Zuckerkonzentration aus der Beobachtung von $\varepsilon''$ deutlich weniger fehleranfällig als die Beobachtung von $\varepsilon'$ bzw. dessen Niveauänderungen.

Die Theorie der Cole-Cole Relaxation ist allerdings eine lineare Theorie, was bedeutet, dass der Verlauf der komplexen Dielektrizitätskonstante $\varepsilon''$ als Funktion der Frequenz von der Stärke der elektromagnetischen Felder unabhängig ist. Dabei handelt es sich aber um eine Näherung im Modell. In der Realität ist der Verlauf abhängig von der Feldstärke und zeigt nichtlineares Verhalten. Beim Cole-Cole Modell geht man wie üblich bei Linearisierungsverfahren von einer Kleinsignalaussteuerung der Felder aus und ignoriert damit nichtlineare Effekte.

Die Sende-Empfangsanordnung 106 kann ferner ausgebildet sein, eine Relaxationszeitkonstante $\tau$ des Blutbildparameters in Abhängigkeit von der Frequenz mit der größeren oder maximalen Verlustgröße zu bestimmen. Weiter kann die Sende-Empfangsanordnung 106 dazu ausgebildet sein, den Blutbildparameter, wie die Glukosekonzentration im Blut, in Abhängigkeit der bestimmten Relaxationszeitkonstanten $\tau$ zu ermitteln.

Gemäß einer Ausführungsform ist die Sende-Empfangsanordnung 106 insbesondere dazu ausgebildet, die Relaxationszeitkonstante $\tau$ auf der Basis der Formel:

$$\tau = \frac{1}{2\pi f_A},$$

zu berechnen, wobei $f_A$ die Relaxationsfrequenz bezeichnet, bei welcher die ermittelte Verlustgröße maximal ist.

Vorteilhafterweise kann dann die Sende-Empfangsanordnung 106 ausgebildet sein, die Relaxationsfrequenz zu bestimmen, bei welcher der Imaginärteil $\varepsilon''$ der komplexen Dielektrizitätszahl maximal ist, und die Relaxationszeitkonstante $\tau$ in Abhängigkeit der bestimmten Frequenz zu ermitteln ist. Der Prozessor 108 kann dann die bestimmte Relaxationsfrequenz $f_A$ benutzen, um den Blutbildparameter, beispielsweise die Glukosekonzentration, zu bestimmen.

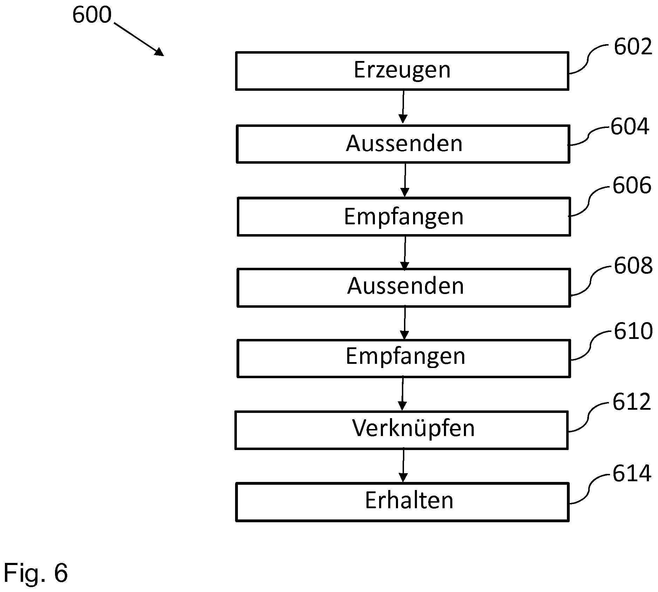

Fig. 6 zeigt eine schematische Darstellung eines Verfahrens 600 zum Erfassen eines Blutbildparameters in einem Blutgefäß 102 gemäß einer Ausführungsform.

Das Verfahren 600 umfasst die folgenden Schritte:

Erzeugen 602 eines Kalibrierungsmesssignals, wobei das Kalibrierungsmesssignal eine Überlagerung aus einem ersten elektrischen Anregungssignal und einem zweiten Anregungssignal umfasst, wobei das erste elektrische Anregungssignal eine höhere Frequenz als das zweite Anregungssignal aufweist, und wobei das zweite Anregungssignal eine höhere elektrische Leistung als das erste elektrische Anregungssignal aufweist;

Aussenden 604 des Kalibrierungsmesssignals in Richtung des Blutgefäßes 102;

Empfangen 606 eines ersten Systemantwortsignals ansprechend auf das Aussenden 604 des Kalibrierungssignals;

Aussenden 608 eines dritten Anregungssignals in Richtung des Blutgefäßes 102;

Empfangen 610 eines zweiten Systemantwortsignals ansprechend auf das Aussenden 608 des dritten Anregungssignals;

Verknüpfen 612 des ersten Systemantwortsignals und des zweiten Systemantwortsignals; und

Erhalten 614 eines Messsignals zur Bestimmung des Blutbildparameters.

Gemäß einer Ausführungsform kann das Verfahren 600 die folgenden Schritte umfassen:

1. Schritt: Auflegen der Erfassungsvorrichtung 100 auf den Körper, z. B. wird eine Antennenanordnung 200 am Handgelenk auf eine obenliegende Ader gelegt;

2. Schritt: Bestimmen einer Referenzkurve auf der Basis einer Mikrowellenmessung mit überlagertem Großsignal. Hierdurch wird die Relaxation behindert/ausgeschaltet;

3. Schritt: Löschen der Polarisation, die beispielsweise durch ein Großsignal erzeugt wurde, durch ein Wechselsignal;

4. Schritt: Messen des eigentlichen Mikrowellensignals ohne Großsignal;

5. Schritt: Berechnen des Messsignals durch Subtraktion der Referenzkurve aus Schritt 2 von der Messkurve aus Schritt 3;

6. Schritt: Bestimmen der Maxima und Minima in der Transmissionsmessung und Ablesen der Relaxationsfrequenzen; und

7. Schritt: Umrechnen der Relaxationsfrequenzen unter Kenntnis der Temperatur des MUT auf die Glukosekonzentration.

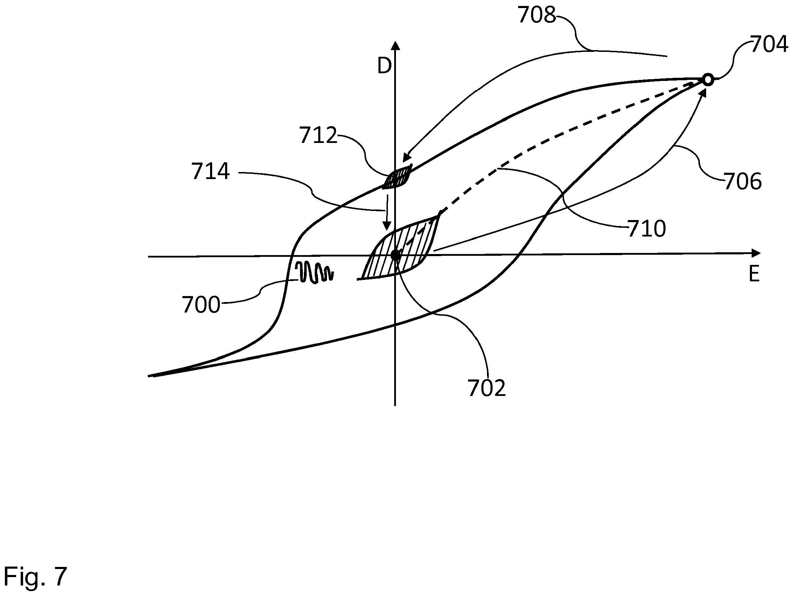

Fig. 7 zeigt eine schematische Darstellung eines Diagrammes zum Auslöschen einer Polarisation von Dipolen in dem Blutgefäß 102, welche durch das von der Erfassungsvorrichtung 100 erzeugte Kalibrierungssignal gemäß einer Ausführungsform verursacht ist.

Gemäß einer Ausführungsform ist der Signalgenerator 104 ausgebildet, das Löschsignal 700, insbesondere als stationäres oder abklingendes Wechselsignal, zu erzeugen. Die Sende-Empfangsanordnung 106 kann ausgebildet sein, das Löschsignal 700 in Richtung des Blutgefäßes 102 auszusenden. Weiterhin kann die Sende-Empfangsanordnung 106 ausgebildet sein, das Löschsignal 700 vor dem Aussenden des Kalibrierungsmesssignals oder nach dem Aussenden des dritten Anregungssignals auszusenden.

In Fig. 7 wird das Verhalten der elektrischen Flussdichte $D$ im Blut als Funktion der elektrischen Feldstärke $E$ eines Großsignales, z. B. eines DC-Signales, gezeigt.

Durch das Einschalten 706 des Großsignals richten sich die elektrischen Dipolmomente des MUTs, z. B. des Blutes in dem Blutgefäß 102, nach dem von Großsignal erzeugten elektrischen Feld aus. Mit steigender elektrischer Feldstärke richten sich immer mehr dieser sogenannten elektrischen Dipolmomente parallel nach dem elektrischen Feld aus (elektrische Polarisation). Bedingt durch die Ausrichtung selbst wird das äußere Feld verstärkt. Es kommt in diesem Zuge zu einer starken Erhöhung der elektrischen Flussdichte sowie des elektrischen Feldes in der Umgebung des MUT. Dieser Vorgang erfolgt nur so lange, bis alle vorhandenen elektrischen Dipolmomente ausgerichtet sind. Sobald das geschehen ist, ist eine Sättigung erreicht, wie es in Fig. 7 durch einen Sättigungspunkt 704 dargestellt ist.

Gemäß einer Ausführungsform ist die Sende-Empfangsanordnung 106 ausgebildet, das dritte Anregungssignal in Richtung des Blutgefäßes 102 auszusenden, wenn der Sättigungspunkt 704 erreicht wird.

Weiterhin kann die Sende-Empfangsanordnung 106 ausgebildet sein, das Großsignal nach dem Empfangen des zweiten Systemantwortsignals auszuschalten 708. Nach dem Ausschalten 708 des Großsignals kann das MUT restliche Effekte der elektrischen Polarisation oder Remanenz 712 aufweisen, d. h. ein Einschalten 706 oder Ausschalten 708 des DC Großsignals stellt keinen perfekten reversiblen Vorgang dar. Daher, um die Erinnerung an das DC Großsignal zu löschen 714 oder die aufgrund des DC Großsignales herbeigeführte Ordnung der elektrischen Dipolmomente wieder in Unordnung zu bringen, kann der Signalgenerator 104 ausgebildet sein, ein abklingendes Signal oder das Löschsignal 700 zu erzeugen. Das Löschsignal 700 kann beispielsweise ein AC Signal sein.

Das Löschen der identischen Ausrichtung der Dipolmomente in dem Blutgefäß 102 entspricht dem Ursprung 702 in dem Flussdichte $D$-$E$ Diagramm, welcher mit dem Sättigungspunkt 704 schematisch durch eine Neukurve 710 verbunden ist.

Weiterhin kann die Sende-Empfangsanordnung 106 ausgebildet sein, das Löschsignal in Richtung des Blutgefäßes 102 auszusenden und ansprechend auf das Aussenden des Löschsignals 700 ein Systemantwortsignal zu empfangen.

Ferner kann der Prozessor 108 ausgebildet sein, ein weiteres Kalibrierungssignal anhand des Systemantwortsignals zu erzeugen. Dieses weitere Kalibrierungssignal kann einer 0 dB Referenzkurve entsprechen, d. h. die Referenzkurve wird bestimmt, wenn kein Großsignal anliegt.

BEZUGSZEICHENLISTE

| | |
|---|---|
| 100 | Erfassungsvorrichtung |
| 102 | Blutgefäß |
| 104 | Signalgenerator |
| 104a | Schalter |
| 106 | Sende-Empfangsanordnung |
| 106a | Netzwerkanalysator |
| 108 | Prozessor |
| 110 | Kondensator |
| 110a | Kondensator |
| 112 | Bias-T |
| 114 | Tor |
| 114a | Tor |
| | |
| 200 | Antennenanordnung / Koppelvorrichtung |
| 202 | Arm |

| 202a | Ader |
| *204 | Leitungsanordnung |
| 300a | Reflexionsmessung |
| 300b | Transmissionsmessung |
| 302a | Welle |
| 302b | Welle |
| 302c | Welle |
| 400 | Darstellung |
| 402 | Impedanz |
| 402a | Impedanz |
| 408 | Wellen |
| 600 | Verfahren |
| 602 | Erzeugen |
| 604 | Aussenden |
| 606 | Empfangen |
| 608 | Aussenden |
| 610 | Empfangen |
| 612 | Verknüpfen |
| 614 | Erhalten |
| 700 | Löschsignal |
| 702 | Ursprung |
| 704 | Sättigungspunkt |
| 706 | Einschalten |
| 708 | Ausschalten |
| 710 | Neukurve |
| 712 | Remanenz |
| 714 | Löschen |

**Patentansprüche**

1. Erfassungsvorrichtung (100) zum Erfassen einer Blutzuckerkonzentration in einem Blutgefäß (102) mit:

   einem Signalgenerator (104), welcher ausgebildet ist, ein erstes elektrisches Anregungssignal mit einer Frequenz zwischen 1 MHz bis 100 GHz und ein zweites elektrisches Anregungssignal, zu erzeugen, wobei der Signalgenerator (104) ausgebildet ist,
   ein Kalibrierungsmesssignal zu erzeugen, wobei das Kalibrierungsmesssignal eine Überlagerung aus dem ersten Anregungssignal und dem zweiten Anregungssignal umfasst,

   - wobei das erste Anregungssignal eine höhere Frequenz als das zweite Anregungssignal aufweist und/oder das zweite Anregungssignal ein Gleichsignal ist und/oder
   - wobei das zweite Anregungssignal eine höhere Leistung als das erste Anregungssignal aufweist;

   einer Sende-Empfangsanordnung (106), welche ausgebildet ist, das Kalibrierungsmesssignal in Richtung des Blutgefäßes (102) auszusenden und ansprechend auf das Aussenden des Kalibrierungsmesssignals ein erstes Systemantwortsignal zu empfangen;
   wobei die Sende-Empfangsanordnung (106) ausgebildet ist, ein drittes Anregungssignal in Richtung des Blutgefäßes (102) auszusenden und ansprechend auf das Aussenden des dritten Anregungssignals ein zweites Systemantwortsignal zu empfangen; und
   einem Prozessor (108), welcher ausgebildet ist, das erste Systemantwortsignal und das zweite Systemantwortsignal zu verknüpfen, um ein Messsignal zur Bestimmung der Blutzuckerkonzentration zu erhalten,
   **gekennzeichnet dadurch, dass** der Signalgenerator (104) ausgebildet ist, ein Löschsignal (700), insbesondere ein stationäres oder abklingendes Wechselsignal, zum Löschen einer durch das Kalibrierungsmesssignal verursachten Polarisation von Dipolen in dem Blutgefäß (102) zu erzeugen, wobei die Sende-Empfangsanordnung (106) ausgebildet ist, das Löschsignal (700) in Richtung des Blutgefäßes (102) auszusenden.

2. Erfassungsvorrichtung (100) nach Anspruch 1, wobei die Sende-Empfangsanordnung (106) eine Antennenanordnung (200) umfasst, welche ausgebildet ist, das elektromagnetische Feld des Kalibrierungsmesssignals oder des dritten Anregungssignals auszusenden.

3. Erfassungsvorrichtung (100) nach Anspruch 2, wobei die Antennenanordnung (200) eine elektrische Leitungsanordnung (204) oder einen halboffenen Wellenleiter, insbesondere einen geschlitzten Hohlleiter, oder eine Mikrostreifenleitung, umfasst.

4. Erfassungsvorrichtung (100) nach einem der vorstehenden Ansprüche, mit einem Armband, wobei zumindest die Sende-Empfangsanordnung (106) oder die Erfassungsvorrichtung (100) in einem Armband integriert ist.

5. Erfassungsvorrichtung (100) nach einem der vorstehenden Ansprüche, wobei der Prozessor (108) ausgebildet ist, eine Differenz aus dem ersten Systemantwortsignal und dem zweiten Systemantwortsignal zu bilden, um das Messsignal zu erhalten.

6. Erfassungsvorrichtung (100) nach einem der vorstehenden Ansprüche, wobei der Signalgenerator (104) ausgebildet ist, das erste Anregungssignal als ein Kleinsignal und das zweite Anregungssignal als ein Großsignal zu erzeugen.

7. Erfassungsvorrichtung (100) nach einem der vorstehenden Ansprüche, wobei der Signalgenerator (104) ausgebildet ist, das erste Anregungssignal als ein hochfrequentes Signal und das zweite Anregungssignal als ein niederfrequentes Signal, insbesondere als ein Gleichsignal, zu erzeugen.

8. Erfassungsvorrichtung (100) nach einem der vorstehenden Ansprüche, wobei der Signalgenerator (104) ausgebildet ist, das erste Anregungssignal mit einer Frequenz bis 100 GHz, insbesondere zwischen 1 MHz bis 100 GHz, zu erzeugen.

9. Erfassungsvorrichtung (100) nach einem der vorstehenden Ansprüche, wobei der Signalgenerator (104) ausgebildet ist, das erste Anregungssignal dem zweiten Anregungssignal zu überlagern, um das Kalibrierungsmesssignal zu erzeugen.

10. Erfassungsvorrichtung (100) nach einem der vorstehenden Ansprüche, wobei die Sende-Empfangsanordnung (106) ausgebildet ist, das erste Systemantwortsignal und das zweite Systemantwortsignal durch eine S-Parametermessung, insbesondere einer Transmissionsmessung (300b) und/oder einer Reflexionsmessung (300a), zu erfassen.

11. Erfassungsvorrichtung (100) nach Anspruch 10, wobei der Signalgenerator (104) ausgebildet ist, das erste Systemantwortsignal und das zweite Systemantwortsignal basierend auf einer Messung eines Vorwärts-Transmissionsfaktors ($S_{21}$) und/oder eines Eingangs-Reflexionsfaktors ($S_{11}$) zu bestimmen.

12. Erfassungsvorrichtung (100) nach einem der vorstehenden Ansprüche, wobei der Prozessor (108) ausgebildet ist, die Blutzuckerkonzentration auf der Basis des Messsignals zu bestimmen.

13. Erfassungsvorrichtung (100) nach einem der vorstehenden Ansprüche, wobei der Prozessor (108) oder die Sende-Empfangsanordnung (106) ausgebildet ist, zur Bestimmung der Blutzuckerkonzentration eine komplexen Dielektrizitätszahl $\varepsilon$ zu bestimmen, wobei der Realteil $\varepsilon'$ der komplexen Dielektrizitätszahl $\varepsilon$ im Wesentlichen die Polarisierbarkeit eines Stoffes im Blut abbildet, und der Imaginärteil $\varepsilon''$ dessen Verluste abbildet.

14. Erfassungsvorrichtung (100) nach Anspruch 13, wobei der Prozessor (108) oder die Sende-Empfangsanordnung (106) ausgebildet ist, zur Bestimmung der Blutzuckerkonzentration eine Relaxationszeitkonstante ($\tau$) auf der Basis der Formel:

$$\tau = \frac{1}{2\pi f_A},$$

zu berechnen, wobei $f_A$ eine Relaxationsfrequenz bezeichnet, bei welcher der Imaginärteil $\varepsilon''$ der komplexen Dielektrizitätszahl maximal ist, und wobei der Prozessor (108) oder die Sende-Empfangsanordnung (106) ausgebildet ist, die Blutzuckerkonzentration, beispielsweise die Glukosekonzentration im Blut, in Abhängigkeit von der bestimmten Relaxationszeitkonstanten $\tau$ zu ermitteln.

15. Erfassungsvorrichtung (100) nach einem der vorstehenden Ansprüche, wobei die Sende-Empfangsanordnung (106) ausgebildet ist, das Löschsignal (700) vor oder nach dem Aussenden des Kalibrierungsmesssignals oder nach dem Aussenden des dritten Anregungssignals auszusenden.

16. Verfahren (600) zum Erfassen einer Blutzuckerkonzentration in einem Blutgefäß (102), wobei das Verfahren (600) die folgenden Schritte umfasst:

Erzeugen (602) eines Kalibrierungsmesssignals, wobei das Kalibrierungsmesssignal eine Überlagerung aus einem ersten Anregungssignal und einem zweiten Anregungssignal umfasst, wobei das erste Anregungssignal ein elektrisches Anregungssignal mit einer Frequenz zwischen 1 MHz bis 100 GHz ist und eine höhere Frequenz als das zweite Anregungssignal aufweist und/oder wobei das zweite Anregungssignal ein Gleichsignal ist und/oder wobei das zweite Anregungssignal eine höhere Leistung als das erste elektrische Anregungssignal aufweist;
Aussenden (604) des Kalibrierungsmesssignals in Richtung des Blutgefäßes (102);
Empfangen (606) eines ersten Systemantwortsignals ansprechend auf das Aussenden des Kalibrierungsmesssignals;
Aussenden (608) eines dritten elektrischen Anregungssignals in Richtung des Blutgefäßes (102);
Empfangen (610) eines zweiten Systemantwortsignals ansprechend auf das Aussenden des dritten Anregungssignals;
Verknüpfen (612) des ersten Systemantwortsignals und des zweiten Systemantwortsignals mittels eines Prozessors (108) um ein Messsignal zur Bestimmung der Blutzuckerkonzentration zu erhalten (614), **gekennzeichnet dadurch, dass** das Verfahren (600) ferner den folgenden Schritt umfasst:
Aussenden eines elektrischen Löschsignals (700) vor und/oder nach dem Aussenden (604) des Kalibrierungsmesssignals und/oder nach dem Aussenden (608) des dritten Anregungssignals, zum Löschen einer Polarisation von Dipolen in dem Blutgefäß.

17. Digitales Speichermedium, insbesondere in Form einer Diskette, CD oder DVD oder EPROM, mit elektronisch auslesbaren Steuersignalen, konfiguriert, um auf einer Erfassungsvorrichtung nach Anspruch 1 ein Verfahren gemäß Anspruch 16 auszuführen.

18. Computerprogramm-Produkt mit einem auf einem maschinenlesbaren Träger gespeicherten Programmcode, um auf einer Erfassungsvorrichtung nach Anspruch 1 ein Verfahren gemäß Anspruch 16 auszuführen.

**Claims**

1. A detecting device (100) for detecting a blood sugar concentration in a blood vessel (102), comprising:

   a signal generator (104) designed to generate a first electrical excitation signal having a frequency between 1 MHz to 100 GHz and a second electrical excitation signal, wherein the signal generator (104) is designed to generate a calibration measurement signal, the calibration measurement signal comprising a superimposition of the first excitation signal and the second excitation signal,

   - wherein the first excitation signal has a higher frequency than the second excitation signal and/or the second excitation signal is a direct signal and/or
   - wherein the second excitation signal has a higher power than the first excitation signal;

   a transceiver arrangement (106) designed to emit the calibration measurement signal in the direction of the blood vessel (102) and to receive a first system response signal in response to the emission of the calibration measurement signal; wherein the transceiver arrangement (106) is designed to emit a third excitation signal in the direction of the blood vessel (102) and to receive a second system response signal in response to the emission of the third excitation signal; and a processor (108) designed to link the first system response signal and the second system response signal in order to obtain a measurement signal for determining the blood sugar concentration,
   **characterized in that**
   the signal generator (104) is designed to generate a clear signal (700), in particular a stationary or subsiding alternating signal, for clearing a polarization of dipoles in the blood vessel (102) caused by the calibration measurement signal, the transceiver arrangement (106) being designed to emit the clear signal (700) in the direction of the blood vessel (102).

2. The detecting device (100) according to claim 1, wherein the transceiver arrangement (106) comprises an antenna arrangement (200) designed to emit the electromagnetic field of the calibration measurement signal or of the third excitation signal.

3. The detecting device (100) according to claim 2, wherein the antenna arrangement (200) comprises an electrical line arrangement (204) or a semi-open waveguide, in particular a slotted waveguide, or a microstrip line.

4. The detecting device (100) according to anyone of the preceding claims, with a bracelet, wherein at least the transceiver arrangement (106) or the detecting device (100) is integrated in a bracelet.

5. The detecting device (100) according to anyone of the preceding claims, wherein the processor (108) is designed to form a difference between the first system response signal and the second system response signal in order to obtain the measurement signal.

6. The detecting device (100) according to anyone of the preceding claims, wherein the signal generator (104) is designed to generate the first excitation signal as a small signal and the second excitation signal as a large signal.

7. The detecting device (100) according to anyone of the preceding claims, wherein the signal generator (104) is designed to generate the first excitation signal as a high-frequency signal and the second excitation signal as a low-frequency signal, in particular as a direct signal.

8. The detecting device (100) according to anyone of the preceding claims, wherein the signal generator (104) is designed to generate the first excitation signal with a frequency of up to 100 GHz, in particular between 1 MHz to 100 GHz.

9. The detecting device (100) according to anyone of the preceding claims, wherein the signal generator (104) is designed to superimpose the first excitation signal on the second excitation signal in order to generate the calibration measurement signal.

10. The detecting device (100) according to anyone of the preceding claims, wherein the transceiver arrangement (106) is designed to detect the first system response signal and the second system response signal using an S-parameter measurement, in particular a transmission measurement (300b) and/or a reflection measurement (300a).

11. The detecting device (100) according to claim 10, wherein the signal generator (104) is designed to determine the first system response signal and the second system response signal based on a measurement of a forward transmission factor ($S_{21}$) and/or an input reflection factor ($S_{11}$).

12. The detecting device (100) according to anyone of the preceding claims, wherein the processor (108) is designed to determine the blood sugar concentration based on the measurement signal.

13. The detecting device (100) according to anyone of the preceding claims, wherein the processor (108) or the transceiver arrangement (106) is designed to determine a complex dielectric constant $\varepsilon$, for the determination of the blood sugar concentration, wherein the real part $\varepsilon'$ of the complex dielectric constant $\varepsilon$ substantially depicts the polarizability of a substance in the blood, and the imaginary part $\varepsilon''$ depicts its losses.

14. The detecting device (100) according to claim 13, wherein the processor (108) or the transceiver arrangement (106) is designed to calculate a relaxation time constant ($\tau$), for the determination of the blood sugar concentration, based on the formula:

$$\tau = \frac{1}{2\pi f_A},$$

wherein $f_A$ denotes a relaxation frequency at which the imaginary part $\varepsilon''$ of the complex dielectric constant is maximal, and wherein the processor (108) or the transceiver arrangement (106) is designed to determine the blood sugar concentration, for example the glucose concentration in the blood, depending on the determined relaxation time constant $\tau$.

15. The detecting device (100) according to anyone of the preceding claims, wherein the transceiver arrangement (106) is designed to emit the clear signal (700) prior to or after emitting the calibration measurement signal or after emitting the third excitation signal.

16. A method (600) for detecting a blood sugar concentration in a blood vessel (102), the method (600) comprising the steps of:

generating (602) a calibration measurement signal, the calibration measurement signal comprising a superimposition of a first excitation signal and a second excitation signal, wherein the first excitation signal is an electrical excitation signal with a frequency between 1 MHz to 100 GHz and has a higher frequency than the second excitation signal and/or wherein the second excitation signal is a direct signal and/or wherein the second excitation signal has a higher power than the first electrical excitation signal;
emitting (604) the calibration measurement signal in the direction of the blood vessel (102);
receiving (606) a first system response signal in response to the emission of the calibration measurement signal;
emitting (608) a third electrical excitation signal in the direction of the blood vessel (102);
receiving (610) a second system response signal in response to the emission of the third excitation signal;
linking (612) the first system response signal and the second system response signal by means of a processor (108) in order to obtain a measurement signal for determining the blood sugar concentration (614),
**characterized in that** the method (600) further comprises the step of:
emitting an electrical clear signal (700) prior and/or after emitting (604) the calibration measurement signal and/or after emitting (608) the third excitation signal, for clearing polarization of dipoles in the blood vessel.

17. A digital storage medium, in particular in the form of a floppy disk, CD or DVD or EPROM, with electronically readable control signals, configured to perform a method according to claim 16 on a suitable detecting device.

18. A computer program product with a program code saved on a machine-readable carrier for performing a method according to claim 16 on a suitable detecting device.

**Revendications**

1. Un dispositif de détection (100) pour détecter une concentration de sucre dans le sang dans un vaisseau sanguin (102), comprenant:

· un générateur de signaux (104) conçu pour générer un premier signal d'excitation électrique ayant une fréquence comprise entre 1 MHz et 100 GHz ainsi qu'un second signal d'excitation électrique, le générateur de signaux (104) étant conçu pour générer un signal de mesure d'étalonnage, le signal de mesure d'étalonnage présentant une superposition du premier signal d'excitation et du second signal d'excitation,

- où le premier signal d'excitation a une fréquence plus élevée que le second signal d'excitation et/ou où le second signal d'excitation est un signal continu et/ou
- où le second signal d'excitation signal a une puissance plus élevée que le premier signal d'excitation;

· un émetteur-récepteur (106) conçu pour émettre le signal de mesure d'étalonnage en direction du vaisseau sanguin (102) et pour recevoir un premier signal de réponse du système en réponse à l'émission du signal de mesure d'étalonnage; où l'émetteur-récepteur (106) est conçu pour émettre un troisième signal d'excitation en direction du vaisseau sanguin (102) et pour recevoir un second signal de réponse du système en réponse à l'émission du troisième signal d'excitation; et
· un processeur (108) conçu pour mettre en relation le premier signal de réponse du système et le second signal de réponse du système afin d'obtenir un signal de mesure permettant de déterminer la concentration de sucre dans le sang,

**caractérisé en ce que**
le générateur de signaux (104) est conçu pour générer un signal de suppression (700), notamment un signal alternatif stationnaire ou décroissant, afin de supprimer une polarisation de dipôles dans le vaisseau sanguin (102) causée par le signal de mesure d'étalonnage, l'émetteur-récepteur (106) étant conçu pour émettre le signal de suppression (700) en direction du vaisseau sanguin (102).

2.  Le dispositif de détection (100) selon la première revendication, où l'émetteur-récepteur (106) comprend un agencement d'antennes (200) conçu pour émettre le champ magnétique du signal de mesure d'étalonnage ou du troisième signal d'excitation.

3.  Le dispositif de détection (100) selon la revendication 2, où l'agencement d'antennes (200) comprend un agencement de conduits électriques (204) ou un guide d'ondes semi-ouvert, notamment un guide d'ondes à fentes, ou un conduit microbande.

4.  Le dispositif de détection (100) selon l'une quelconque des revendications précédentes, avec un bracelet, où au moins l'émetteur-récepteur (106) ou le dispositif de détection (100) est intégré dans un bracelet.

5.  Le dispositif de détection (100) selon l'une quelconque des revendications précédentes, où le processeur (108) est conçu pour former une différence entre le premier signal de réponse du système et le second signal de réponse du système afin d'obtenir le signal de mesure.

6.  Le dispositif de détection (100) selon l'une quelconque des revendications précédentes, où le générateur de signaux (104) est conçu pour générer le premier signal d'excitation sous la forme d'un petit signal et le second signal d'excitation sous la forme d'un grand signal.

7.  Le dispositif de détection (100) selon l'une quelconque des revendications précédentes, où le générateur de signaux (104) est conçu pour générer le premier signal d'excitation sous la forme d'un signal à haute fréquence et le second signal d'excitation sous la forme d'un signal à basse fréquence, notamment sous la forme d'un signal continu.

8.  Le dispositif de détection (100) selon l'une quelconque des revendications précédentes, où le générateur de signaux (104) est conçu pour générer le premier signal d'excitation avec une fréquence allant jusqu'à 100 GHz, notamment entre 1 MHz et 100 GHz.

9.  Le dispositif de détection (100) selon l'une quelconque des revendications précédentes, où le générateur de signaux (104) est conçu pour superposer le premier signal d'excitation sur le second signal d'excitation afin de générer le signal de mesure d'étalonnage.

10. Le dispositif de détection (100) selon l'une quelconque des revendications précédentes, où l'émetteur-récepteur (106) est conçu pour détecter le premier signal de réponse du système ainsi que le second signal de réponse du système en utilisant une mesure de paramètres S, notamment une mesure de transmission (300b) et/ou une mesure de réflexion (300a).

11. Le dispositif de détection (100) selon la revendication 10, où le générateur de signaux (104) est conçu pour déterminer le premier signal de réponse du système ainsi que le second signal de réponse du système sur la base d'une mesure d'un facteur de transmission aval ($S_{21}$) et/ou d'un facteur de réflexion d'entrée ($S_{11}$).

12. Le dispositif de détection (100) selon l'une quelconque des revendications précédentes, où le processeur (108) est conçu pour déterminer la concentration de sucre dans le sang sur la base du signal de mesure.

13. Le dispositif de détection (100) selon l'une quelconque des revendications précédentes, où le processeur (108) ou l'émetteur-récepteur (106) est conçu pour déterminer une constante diélectrique complexe $\varepsilon$, pour la détermination de la concentration de sucre dans le sang, la partie réelle $\varepsilon'$ de la constante diélectrique complexe $\varepsilon$ représentant essentiellement la polarisabilité d'une substance dans le sang, et la partie imaginaire $\varepsilon''$ représentant ses pertes.

14. Le dispositif de détection (100) selon la revendication 13, où le processeur (108) ou l'émetteur-récepteur (106) est conçu pour calculer une constante de temps de relaxation ($\tau$), pour la détermination de la concentration de sucre dans le sang, sur la base de la formule:

$$\tau = \frac{1}{2\pi f_A},$$

où $f_A$ désigne une fréquence de relaxation pour laquelle la partie imaginaire $\varepsilon''$ de la constante diélectrique complexe est maximale, et où le processeur (108) ou l'émetteur-récepteur (106) est conçu pour déterminer la concentration de sucre dans le sang, par exemple la concentration de glucose dans le sang, en fonction de la constante de temps de relaxation déterminée $\tau$.

15. Le dispositif de détection (100) selon l'une quelconque des revendications précédentes, où l'émetteur-récepteur (106) est conçu pour émettre le signal de suppression (700) avant ou après l'émission su signal de mesure d'étalonnage ou après l'émission du troisième signal d'excitation.

16. Un procédé (600) pour détecter une concentration de sucre dans le sang dans un vaisseau sanguin (102), le procédé (600) comprenant les étapes suivantes:

    . la génération (602) d'un signal de mesure d'étalonnage, le signal de mesure d'étalonnage comprenant une superposition d'un premier signal d'excitation et d'un second signal d'excitation, où le premier signal d'excitation est un signal d'excitation électrique ayant une fréquence comprise entre 1 MHz et 100 GHz et une fréquence supérieure au second signal et/ou où le second signal d'excitation est un signal continu et/ou où le second signal d'excitation a une puissance supérieure au premier signal d'excitation électrique;
    . l'émission (604) du signal de mesure d'étalonnage en direction du vaisseau sanguin (102);
    . la réception (606) d'un premier signal de réponse du système en réponse à l'émission du signal de mesure d'étalonnage;
    . l'émission (608) d'un troisième signal d'excitation électrique en direction du vaisseau sanguin (102);
    . la réception (610) d'un second signal de réponse du système en réponse à l'émission du troisième signal d'excitation;
    . la mise en relation (612) du premier signal de réponse du système et du second signal de réponse du système au moyen d'un processeur (108) afin d'obtenir un signal de mesure pour la détermination de la concentration de sucre dans le sang (614),

**caractérisé en ce que** le procédé (600) comprend en outre l'étape suivante:
    . l'émission d'un signal de suppression électrique (700) avant et/ou après l'émission (604) du signal de mesure d'étalonnage et/ou après l'émission (608) du troisième signal d'excitation, pour supprimer une polarisation de dipôles dans le vaisseau sanguin.

17. Un support de stockage numérique, notamment sous la forme d'une disquette, d'un CD ou d'un DVD ou d'un EPROM, avec des signaux de commande lisibles électroniquement, configuré pour exécuter un procédé selon la revendication 16 sur un dispositif de détection approprié à cet effet.

18. Un produit de programme informatique ayant un code de programme stocké sur un support lisible par machine pour exécuter un procédé selon la revendication 16 sur un dispositif de détection approprié à ce effet.

EP 3 664 707 B1

100

104

Signalgenerator

106

102

Sende-
Empfangsanordnung

Blutgefäß

108

Prozessor

Fig. 1

100

114a

204

290

202

110a

106a

114

202a

2

1

112

110

104a

104

Fig. 2

Fig. 3a

EP 3 664 707 B1

Fig. 3b

400

102

408

402

MUT

$\varepsilon_{r MUT}, \mu_{r MUT}$

$Z_{L1}$

$Z_{L1}$

402a

$Z_2(\varepsilon_{r MUT}, \mu_{r MUT}) \; ; \; \gamma_2(\varepsilon_{r MUT}, \mu_{r MUT})$

114

114a

Fig. 4

Fig. 5

EP 3 664 707 B1

EP 3 664 707 B1

600

```
┌─────────────────────────┐
│        Erzeugen         │──── 602
└─────────────────────────┘
             │
             ▼
┌─────────────────────────┐
│        Aussenden        │──── 604
└─────────────────────────┘
             │
             ▼
┌─────────────────────────┐
│        Empfangen        │──── 606
└─────────────────────────┘
             │
             ▼
┌─────────────────────────┐
│        Aussenden        │──── 608
└─────────────────────────┘
             │
             ▼
┌─────────────────────────┐
│        Empfangen        │──── 610
└─────────────────────────┘
             │
             ▼
┌─────────────────────────┐
│       Verknüpfen        │──── 612
└─────────────────────────┘
             │
             ▼
┌─────────────────────────┐
│        Erhalten         │──── 614
└─────────────────────────┘
```

Fig. 6

Fig. 7

EP 3 664 707 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CN 104856690 A

- CN 105342627 A

**In der Beschreibung aufgeführte Nicht-Patentliteratur**

- **VON BUFORD RANDAL JEAN et al.** A microwave frequency sensor for non-invasive blood-glucose measuremen. *SAS 2008 - IEEE Sensors Applications Symposium, Atlanta, GA,* 12. Februar 2008

- Calibration methodology for a microwave non-invasive glucose sensor. **VON M. MCCLUNG.** Master's Thesis. Baylor University, Mai 2008